# EUROPEAN PATENT SPECIFICATION

(11) **EP 2 871 686 B1**
(45) Date of publication and mention of the grant of the patent: **21.08.2019**
(21) Application number: 13812630.5
(22) Date of filing: 03.07.2013
(51) Int. Cl.: H01L 51/54, C07D 471/04, C09K 11/06

(54) **LIGHT EMITTING ELMENT MATERIAL AND LIGHT EMITTING ELEMENT**
MATERIAL FÜR EIN LICHTEMITTIERENDES ELEMENT UND LICHTEMITTIERENDES ELEMENT
MATÉRIAU D'ÉLÉMENT ÉLECTROLUMINESCENT ET ÉLÉMENT ÉLECTROLUMINESCENT

(30) Priority: 05.07.2012 JP 2012151040
(43) Date of publication of application: 13.05.2015
(73) Proprietor: Toray Industries, Inc., Tokyo 103-8666 (JP)
(72) Inventor: ICHIHASHI, Yasunori, Otsu-shi Shiga 520-8558 (JP); TANAKA, Daisaku, Otsu-shi Shiga 520-8558 (JP); IKEDA, Takeshi, Otsu-shi Shiga 520-8558 (JP); ARAI, Takeshi, Otsu-shi Shiga 520-8558 (JP); IKEDA, Atsushi, Otsu-shi Shiga 520-8558 (JP); TOMINAGA, Tsuyoshi, Otsu-shi Shiga 520-8558 (JP)
(74) Representative: Hoefer & Partner Patentanwälte mbB
(86) International application number: PCT/JP2013/068242
(87) International publication number: WO 2014/007287

(56) References cited:
- EP-A1- 2 674 418
- EP-A2- 1 617 493
- WO-A1-2011/132683
- WO-A1-2011/132684
- WO-A1-2012/030145
- WO-A1-2012/108388
- KR-A- 20120 020 816
- PAYAL TYAGI ET AL: "Solution Processable Indoloquinoxaline Derivatives Containing Bulky Polyaromatic Hydrocarbons: Synthesis, Optical Spectra, and Electroluminescence", THE JOURNAL OF ORGANIC CHEMISTRY, vol. 76, no. 11, 3 June 2011 (2011-06-03), pages 4571-4581, XP055171236, ISSN: 0022-3263, DOI: 10.1021/jo2004764
- PAYAL TYAGI ET AL.: 'Solution Processable Indoloquinoxaline erivatives Containing Bulky Polyaromatic Hydrocarbons: Synthesis, Optical Spectra, and Electroluminescence' THE JOURNAL OF ORGANIC CHEMISTRY vol. 76, no. 11, 03 May 2011, pages 4571 - 4581, XP055171236
- K.R.J.THOMAS ET AL.: 'Synthesis, Spectra, and Theoretical Investigations of the Triarylamines Based on 6H-Indolo[2,3-b]quinoxaline' THE JOURNAL OF ORGANIC CHEMISTRY vol. 75, no. 23, 11 May 2010, pages 8100 - 8111, XP055174266

## Description

### TECHNICAL FIELD

The present invention relates to a light-emitting device capable of converting electric energy into light, and a material to be used for the same. The present invention is capable of being used for areas such as display devices, flat-panel displays, backlight, lighting, interior design, labels, signboards, electrophotography machines, and light signal generators.

### BACKGROUND ART

Researches on an organic thin-film light-emitting device in which electrons injected from a cathode and holes injected from an anode emit light when they are recombined in an organic fluorescent body held by both electrodes have been actively conducted in recent years. This light-emitting device is characteristic for high luminance light emission in the form of a thin type and under a low driving voltage, and multicolor light emission due to selection of a fluorescent material, and has been paid attention.

Such researches have undergone many studies for practical use since C. W. Tang et al. of Kodak Co., Ltd. showed that an organic thin-film device emits light at high luminance, and organic thin-film light-emitting devices have steadily come into practical use as they have been employed in main displays of mobile phones, and the like. However, there are still many technical problems and, especially, attainment of both increased efficiency and prolonged life of a device is one of the major problems.

For the organic thin-film light-emitting device, it is necessary to satisfy an improvement in luminous efficiency, a reduction in driving voltage and an improvement in durability. Particularly, realization of both luminous efficiency and durable life is a major problem. For example, materials having a fluoranthene skeleton and a nitrogen-containing heterocyclic ring have been developed for improving luminous efficiency and durable life (see, for example, Patent Documents 1 to 3).

WO2012030145 (A) and Payal Tyagi et al ("Solution Processable Indoloquinoxaline Derivatives Containing Bulky Polyaromatic Hydrocarbons: Synthesis, Optical Spectra, and Electroluminescence",THE JOURNAL OF ORGANIC CHEMISTRY, vol. 76, no. 11, 3 June 2011 (2011-06-03), pages 4571-4581) disclose fluoranthene based materials for use in OLEDs.

### PRIOR ART DOCUMENT

### PATENT DOCUMENTS

Patent Document 1: WO 2010/114264
Patent Document 2: WO 2007/100010
Patent Document 3: WO 2004/053019

### SUMMARY OF THE INVENTION

### PROBLEMS TO BE SOLVED BY THE INVENTION

However, conventional technologies were difficult to reduce the driving voltage of a device sufficiently, and even if they had been able to reduce the driving voltage, the luminous efficiency and the durable life of a device were insufficient. Thus, technologies capable of realizing all of high luminous efficiency, low driving voltage and durable life have not been found yet.

An object of the present invention is to solve such problems with the conventional technologies and provide an organic thin-film light-emitting device that has improved all of luminous efficiency, driving voltage and durable life.

### SOLUTIONS TO THE PROBLEMS

The present invention provides a light-emitting device material including a compound represented by the following general formula (1): [Chemical Formula 1]

Ar-L(̵Z)ₙ (1)

wherein Z is represented by the following general formula (2), and Ar is represented by the following general formula (3) ; L is a substituted or unsubstituted arylene group; n is 1 or 2; and when n is 2, two Zs may be the same or different 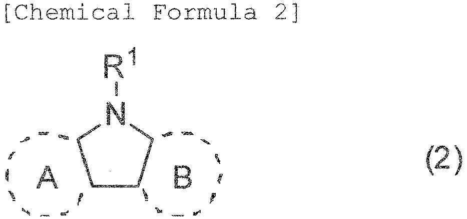 wherein ring A and ring B each represent a substituted or unsubstituted benzene ring, a substituted or unsubstituted fused aromatic hydrocarbon ring, a substituted or unsubstituted monocyclic aromatic heterocyclic ring, or a substituted or unsubstituted fused aromatic heterocyclic ring, with the proviso that at least one of atoms that form ring A and ring B is electron-accepting nitrogen; a substituent that ring A and ring B optionally have, and R¹ are each selected from the group consisting of an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, halogen, a carbonyl group, a carboxyl group, an oxycarbonyl group, a carbamoyl group and -P(=O)R²R³; R¹ may be hydrogen; R² and R³ are each an aryl group or a heteroaryl group; or R² and R³ may be fused to form a ring, with the proviso that the group is coupled to L at the position of any of R¹, ring A and ring B; and when n is 2, positions at which two Zs are coupled to L may be the same or different; 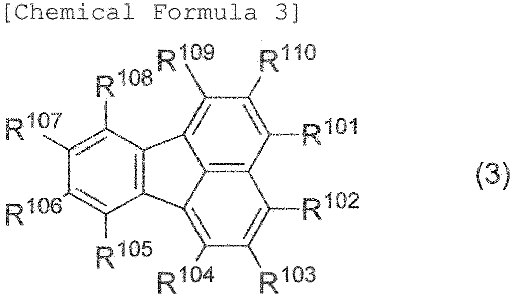 wherein R¹⁰¹ to R¹¹⁰ may be the same or different, and are each selected from the group consisting of hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, halogen, a carbonyl group, a carboxyl group, an oxycarbonyl group and a carbamoyl group; R¹⁰¹ to R¹¹⁰ may form a ring by adjacent substituents, with the proviso that R¹⁰¹ to R¹¹⁰ do not form a benzene ring; and the group is coupled to L at the position of any one of R¹⁰¹ to R¹¹⁰.

### EFFECTS OF THE INVENTION

According to the present invention, there can be provided an organic thin-film light-emitting device that realizes all of luminous efficiency, driving voltage and durable life.

### EMBODIMENTS OF THE INVENTION

The compound represented by the general formula (1) is described in detail below.
[Chemical Formula 4]

Ar-L(̵Z)ₙ (1)

wherein Z is represented by the following general formula (2), and Ar is represented by the following general formula (3) ; L is a substituted or unsubstituted arylene group; n is 1 or 2; and when n is 2, two Zs may be the same or different: 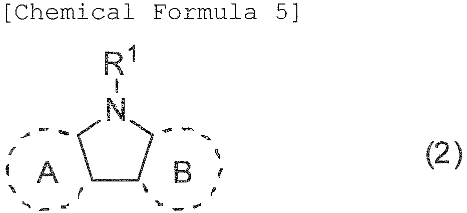 wherein ring A and ring B each represent a substituted or unsubstituted benzene ring, a substituted or unsubstituted fused aromatic hydrocarbon ring, a substituted or unsubstituted monocyclic aromatic heterocyclic ring, or a substituted or unsubstituted fused aromatic heterocyclic ring, with the proviso that at least one of atoms that form ring A and ring B is electron-accepting nitrogen; a substituent that ring A and ring B optionally have, and R¹ are each selected from the group consisting of an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, halogen, a carbonyl group, a carboxyl group, an oxycarbonyl group, a carbamoyl group and -P(=O)R²R³; R¹ may be hydrogen; R² and R³ are each an aryl group or a heteroaryl group; or R² and R³ may be fused to form a ring, with the proviso that the group is coupled to L at the position of any of R¹, ring A and ring B; and when n is 2, positions at which two Zs are coupled to L may be the same or different. 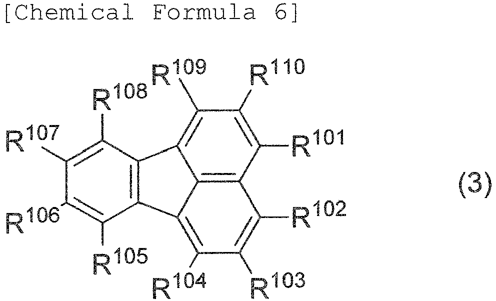 wherein R¹⁰¹ to R¹¹⁰ may be the same or different, and are each selected from the group consisting of hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, halogen, a carbonyl group, a carboxyl group, an oxycarbonyl group and a carbamoyl group; R¹⁰¹ to R¹¹⁰ may form a ring by adjacent substituents, with the proviso that R¹⁰¹ to R¹¹⁰ do not form a benzene ring; and the group is coupled to L at the position of any one of R¹⁰¹ to R¹¹⁰.

In all the groups described above, hydrogen may be heavy hydrogen. The alkyl group denotes a saturated aliphatic hydrocarbon group, such as a methyl group, an ethyl group, a n-propyl group, an isopropyl group, a n-butyl group, a sec-butyl group, or a tert-butyl group, and it may or may not have a substituent. When the alkyl group is substituted, the additional substituent is not particularly limited, examples of the additional substituent may include an alkyl group, an aryl group and a heteroaryl group, and the same holds true in the descriptions below. The number of carbon atoms in the alkyl group is not particularly limited, but from the viewpoints of easy availability and cost, it is preferably within the range of 1 or more and 20 or less, more preferably 1 or more and 8 or less.

The cycloalkyl group denotes a saturated alicyclic hydrocarbon group, such as a cyclopropyl group, a cyclohexyl group, a norbornyl group, or an adamantyl group, and this may or may not have a substituent. The number of carbon atoms in the alkyl group moiety is not particularly limited, but is preferably within the range of 3 or more and 20 or less.

The heterocyclic group denotes an aliphatic ring having an atom other than carbon in the ring, such as a pyran ring, a piperidine ring, or a cyclic amide, and this may or may not have a substituent. The number of carbon atoms in the heterocyclic group is not particularly limited, but is preferably within the range of 2 or more and 20 or less.

The alkenyl group denotes an unsaturated aliphatic hydrocarbon group containing a double bond, such as a vinyl group, an allyl group, or a butadienyl group, and this may or may not have a substituent. The number of carbon atoms in the alkenyl group is not particularly limited, but is preferably within the range of 2 or more and 20 or less.

The cycloalkenyl group denotes an unsaturated alicyclic hydrocarbon group containing a double bond, such as a cyclopentenyl group, a cyclopentadienyl group, or a cyclohexenyl group, and this may or may not have a substituent.

The alkynyl group denotes an unsaturated aliphatic hydrocarbon group containing a triple bond, such as an ethynyl group, and this may or may not have a substituent. The number of carbon atoms in the alkynyl group is not particularly limited, but is preferably within the range of 2 or more and 20 or less.

The alkoxy group denotes a functional group with an aliphatic hydrocarbon group bonded via an ether bond, such as a methoxy group, an ethoxy group, or a propoxy group, and this aliphatic hydrocarbon group may or may not have a substituent. The number of carbon atoms in the alkoxy group is not particularly limited, but is preferably within the range of 1 or more and 20 or less.

The alkylthio group denotes a group in which an oxygen atom of an ether bond in an alkoxy group is substituted with a sulfur atom. The hydrocarbon group of the alkylthio group may or may not have a substituent. The number of carbon atoms in the alkylthio group is not particularly limited, but is preferably within the range of 1 or more and 20 or less.

The aryl ether group denotes a functional group with an aromatic hydrocarbon group bonded via an ether bond, such as a phenoxy group, and the aromatic hydrocarbon group may or may not have a substituent. The number of carbon atoms in the aryl ether group is not particularly limited, but is preferably within the range of 6 or more and 40 or less.

The aryl thioether group denotes a group in which an oxygen atom of an ether bond in an aryl ether group is substituted with a sulfur atom. The aromatic hydrocarbon group in the aryl ether group may or may not have a substituent. The number of carbon atoms in the aryl ether group is not particularly limited, but is preferably within the range of 6 or more and 40 or less.

The aryl group denotes an aromatic hydrocarbon group, such as a phenyl group, a naphthyl group, a biphenyl group, a phenanthryl group, a terphenyl group, a pyrenyl group, or a fluoranthenyl group. The aryl group may or may not have a substituent. The number of carbon atoms in the aryl group is not particularly limited, but is preferably within the range of 6 or more and 40 or less.

The heteroaryl group denotes a cyclic aromatic group having one or a plurality of atoms other than carbon in the ring, such as a furanyl group, a thiophenyl group, a pyridyl group, a quinolinyl group, an isoquinolinyl group, a pyrazinyl group, a pyrimidyl group, a naphthyridyl group, a benzofuranyl group, a benzothiophenyl group, an indolyl group, a dibenzofuranyl group, or a carbazolyl group, and this may be unsubstituted or substituted. The number of carbon atoms in the heteroaryl group is not particularly limited, but is preferably within the range of 2 or more and 30 or less.

The halogen denotes an atom selected from fluorine, chlorine, bromine, and iodine.

The carbonyl group, the carboxyl group, the oxycarbonyl group, the carbamoyl group and the phosphine oxide group may or may not have a substituent. Here, examples of the substituent include an alkyl group, a cycloalkyl group, an aryl group and a heteroaryl group, and these substituents may be further substituted.

The arylene group denotes a divalent or trivalent group derived from an aromatic hydrocarbon group such as a phenyl group, a naphthyl group, or a biphenyl group, and this may or may not have a substituent. When the L in the general formula (1) is an arylene group, the number of nucleus carbon atoms is preferably within the range of 6 or more and 30 or less. Specific examples of the arylene group include a 1, 4-phenylene group, a 1,3-phenylene group, a 1,2-phenylene group, a 4,4'-biphenylene group, a 4,3'-biphenylene group, a 3,3'-biphenylene group, a 1,4-naphthylene group, a 1,5-naphthylene group, a 2,5-naphthylene group, a 2,6-naphthylene group and a 2,7-naphthylene group. A 1,4-phenylene group and a 1,3-phenylene group are more preferable.

The heteroarylene group denotes a divalent or trivalent group derived from an aromatic group having one or a plurality of atoms other than carbon in the ring, such as a pyridyl group, a quinolinyl group, a pyrimidinyl group, a pyrazinyl group, a naphthyridyl group, a dibenzofuranyl group, a dibenzothiophenyl group, or a carbazolyl group, and this may or may not have a substituent. The number of carbon atoms in the heteroarylene group is not particularly limited, but is preferably within the range of 2 to 30.

Examples of the fused aromatic hydrocarbon ring include a naphthalene ring, an azulene ring, an anthracene ring, a phenanthrene ring, a pyrene ring, a chrysene ring, a naphthacene ring, a triphenylene ring, an acenaphthene ring, a coronene ring, a fluorene ring, a fluoranthene ring, a naphthacene ring, a pentacene ring, a perylene ring, a pentaphene ring, a picene ring, a pyranthrene and an anthraanthrene ring. Further, the fused aromatic hydrocarbon ring may have a substituent.

Examples of the monocyclic aromatic heterocyclic ring include a furan ring, a thiophene ring, a pyridine ring, a pyridazine ring, a pyrimidine ring, a pyrazine ring, an oxadiazole ring, a triazole ring, an imidazole ring, a pyrazole ring and a thiazole ring. Further, the monocyclic aromatic heterocyclic ring may have a substituent.

Examples of the fused aromatic heterocyclic ring include a quinoline ring, an isoquinoline ring, a quinoxaline ring, a benzimidazole ring, an indole ring, a benzimidazole ring, a benzothiazole ring, a benzoxazole ring, a quinoxaline ring, a quinazoline ring, a phthalazine ring, a carbazole ring, a carboline ring and a diazacarbazole ring (a ring in which one of carbon atoms of a hydrocarbon ring that forms a carboline ring is further substituted with a nitrogen atom). Further, the fused aromatic heterocyclic ring may have a substituent.

In the compound represented by the above general formula (1), L is a substituted or unsubstituted arylene group. Z represented by the above general formula (2) is coupled to L at the position of any of R¹, ring A and ring B.

Preferably, Z represented by the above general formula (2) is coupled to L at the position of any of ring A and ring B.

The group being coupled to L at the position of any of R¹, ring A and ring B has the following meaning. First, the group being coupled to L at the position of R¹ means that the nitrogen atom coupled to R¹ is directly bonded to L. The group being coupled to L at the position of either ring A or ring B means that for example when ring A is a benzene ring, L is directly bonded to any one of carbon atoms that form the benzene ring.

L is a substituted or unsubstituted arylene group. When L is a substituted or unsubstituted arylene group, conjugation is further expanded to achieve high carrier mobility and high electron-accepting property. As a result, low-voltage driving of the light-emitting device becomes possible, so that luminous efficiency can be improved. Crystallinity can be reduced and the glass transition temperature can be increased, so that stability of the film is improved, and therefore improvement of life becomes possible when the compound is used for the light-emitting device.

In the light-emitting device material of the present invention, n is 1 or 2. That is, the compound represented by the general formula (1) has one or two groups represented by Z, and accordingly crystallinity is reduced and the glass transition temperature is increased, so that stability of the film is improved. n is preferably 1. When n is 1, sublimability and deposition stability are improved.

The light-emitting device material of the present invention has a fluoranthene skeleton. The fluoranthene skeleton has a five-membered ring structure of 5π electron system. When given one electron (when reduced), the five-membered ring structure of 5π electron system turns to a 6π electron system, so that aromatic stabilization occurs (Huckel's rule) . Thus, the five-membered ring structure of 5π electron system shows high electron affinity, and the fluoranthene skeleton according to the present invention also has high electron affinity. Since anthracene and pyrene, generally famous fused ring aromatic skeletons, do not have a five-membered ring structure of 5π electron system, there is no increase in electron affinity resulting from aromatic stabilization due to reduction, and such a phenomenon is a nature specific to a skeleton having a five-membered ring structure of 5π electron system. Thus, when the light-emitting device material of the present invention is used for the light-emitting device, e.g., used for an electron transporting layer, proper electron injection property from the electrode is exhibited, so that the driving voltage of the light-emitting device can be reduced. As a result, luminous efficiency of the light-emitting device can be improved. The light-emitting device material also contributes to an increase in life.

The fluoranthene skeleton has high flatness, so that molecules are well superimposed on one another, and therefore high charge transporting property is achieved. Thus, when the light-emitting device material of the present invention is used for any of the layers that form the light-emitting device, electrons generated from the cathode and holes generated from the anode can be efficiently transported, and therefore the driving voltage of the device can be reduced. As a result, luminous efficiency of the light-emitting device can be improved. The light-emitting device material also contributes to an increase in life.

The fluoranthene skeleton has high stability to charges, so that reduction by electrons and oxidation by holes can be smoothly repeatedly performed. When the light-emitting device material of the present invention is used for the light-emitting device, life can be improved.

In Z represented by the above general formula (2), at least one of atoms that form ring A and ring B is electron-accepting nitrogen. Here, the electron-accepting nitrogen denotes a nitrogen atom which forms a multiple bond with an adjoining atom. Since nitrogen atoms have high electronegativity, the multiple bond has an electron-accepting nature. For this reason, Z having electron-accepting nitrogen has high electron affinity. Thus, when the light-emitting device material of the present invention is used for an emissive layer and an electron transporting layer, proper electron injection property from the electrode is exhibited, so that the driving voltage of the light-emitting device can be reduced. As a result, luminous efficiency of the light-emitting device can be improved. The light-emitting device material also contributes to an increase in life.

In the group represented by Z, the number of electron-accepting nitrogen atoms that form ring A and ring B is preferably 1. When in the group represented by Z, the number of electron-accepting nitrogen atoms that form ring A and ring B is 1, both electron injection property from the electrode and electron injection property to the emissive layer can be realized and thus the driving voltage of the light-emitting device can be reduced when the light-emitting device material is used for the electron transporting layer. As a result, luminous efficiency of the light-emitting device can be improved. On the other hand, when the number of electron-accepting nitrogen atoms is 2 or more, electron injection property to the emissive layer may be deteriorated in the light-emitting device, so that the driving voltage may increase, leading to reduction of luminous efficiency.

The group represented by Z has electron-donating nitrogen. Here, the electron-donating nitrogen denotes a nitrogen atom in which all the bonds with adjacent atoms are single bonds. In the group represented by Z, the nitrogen atom bonded to R¹ is the electron-donating nitrogen. The electron denoting nitrogen has high stability to holes, so that oxidation by holes can be smoothly repeatedly performed. Accordingly, when the light-emitting device material of the present invention is used for the hole transporting layer, life can be improved.

When the light-emitting device material of the present invention has a group of Z, sublimability and deposition stability are improved, crystallinity is reduced, and stability of the film due to a high glass transition temperature is improved. Consequently, when the compound represented by the general formula (1) according to the present invention is used for the light-emitting device, life can be improved.

From the above, the light-emitting device material of the present invention has in the molecule a fluoranthene skeleton and a group represented by Z, and therefore has high electron injection/transporting properties, electrochemical stability, proper sublimability, proper deposition stability, proper film quality and a high glass transition temperature. Thus, when the light-emitting device material of the present invention is used for any of the layers that form the light-emitting device, an organic thin-film light-emitting device having all of high luminous efficiency, low driving voltage and durable life can be provided.

The compound represented by the general formula (1) is preferably a compound represented by the following general formula (4). The compound represented by the general formula (4) is a compound in which the fluoranthene skeleton is substituted at position 3 with a substituent containing Z. In a fluoranthene derivative, when the fluoranthene skeleton is substituted at position 3 with an aromatic substituent, the electronic state thereof is significantly changed, so that conjugation is efficiently expanded, resulting in improvement of charge transporting property. As a result, the light-emitting device can be driven at a lower voltage, so that luminous efficiency can be further improved. Further, since conjugation is expanded, stability to a charge is improved. As a result, when the compound represented by the general formula (4) according to the present invention is used for the light-emitting device, life can be further improved. 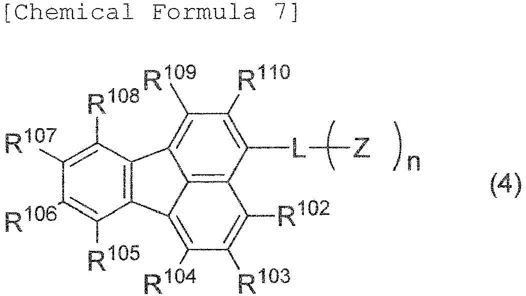 wherein R¹⁰² to R¹¹⁰ may be the same or different, and are each selected from the group consisting of hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, halogen, a carbonyl group, a carboxyl group, an oxycarbonyl group and a carbamoyl group; R¹⁰² to R¹¹⁰ may form a ring by adjacent substituents, with the proviso that R¹⁰¹ to R¹¹⁰ do not form a benzene ring; and L, Z and n are the same as in the general formula (1).

Preferably, R¹⁰² to R¹¹⁰ in the general formula (4) are each selected from the group consisting of hydrogen, an alkyl group, a cycloalkyl group, an aryl group, a heteroaryl group and halogen among the groups described above. When R¹⁰² to R¹¹⁰ are each selected from the group consisting of hydrogen, an alkyl group, a cycloalkyl group, an aryl group, a heteroaryl group and halogen, the glass transition temperature is increased, and thin-film stability is further improved. Since the substituent is hard to be decomposed under a high temperature, heat resistance is further improved. Further, when the substituent is an aryl group or a heteroaryl group, conjugation is expanded, so that electrochemical stability becomes higher, and charge transporting property is improved.

n is preferably 1. When n is 1, sublimability and deposition stability are further improved.

Z is preferably a group represented by any of the following general formulae (5) to (9). When Z is a group represented by any of the following general formulae (5) to (9), high electron mobility and high electron-accepting property are achieved, so that the driving voltage of the light-emitting device can be further reduced. As a result, luminous efficiency of the light-emitting device can be improved. The light-emitting device material also contributes to a further increase in life. 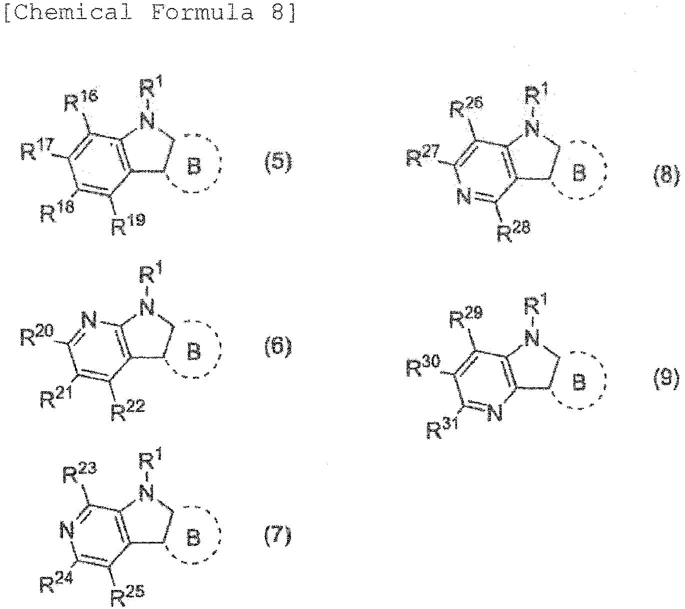 wherein ring B represents a substituted or unsubstituted benzene ring, a substituted or unsubstituted fused aromatic hydrocarbon ring, a substituted or unsubstituted monocyclic aromatic heterocyclic ring, or a substituted or unsubstituted fused aromatic heterocyclic ring, with the proviso that in the case of the general formula (5), ring B is a substituted or unsubstituted monocyclic aromatic heterocyclic ring, or a substituted or unsubstituted fused aromatic heterocyclic ring, and at least one of atoms that form ring B is electron-accepting nitrogen; a substituent that ring B optionally has, and R¹ are the same as in the general formula (2); and R¹⁶ to R³¹ are each selected from the group consisting of hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, halogen, a carbonyl group, a carboxyl group, an oxycarbonyl group, a carbamoyl group and -P(=O)R²R³, with the proviso that the group is coupled to L at the position of any of R¹, R¹⁶ to R¹⁹ and ring B in the case of the general formula (5); at the position of any of R¹, R²⁰ to R²² and ring B in the case of the general formula (6); at the position of any of R¹, R²³ to R²⁵ and ring B in the case of the general formula (7); at the position of any of R¹, R²⁶ to R²⁸ and ring B in the case of the general formula (8); and at the position of any of R¹, R²⁹ to R³¹ and ring B in the case of the general formula (9).

Ring B is preferably a structure represented by any of the following general formulae (10) to (13). When ring B is a structure represented by any of the following general formulae (10) to (13), high carrier mobility and high electron-accepting property are achieved. As a result, further low-voltage driving of the light-emitting device becomes possible, so that luminous efficiency can be further improved. Sublimability and deposition stability are further improved, crystallinity is further reduced, and stability of the film due to a high glass transition temperature is further improved. 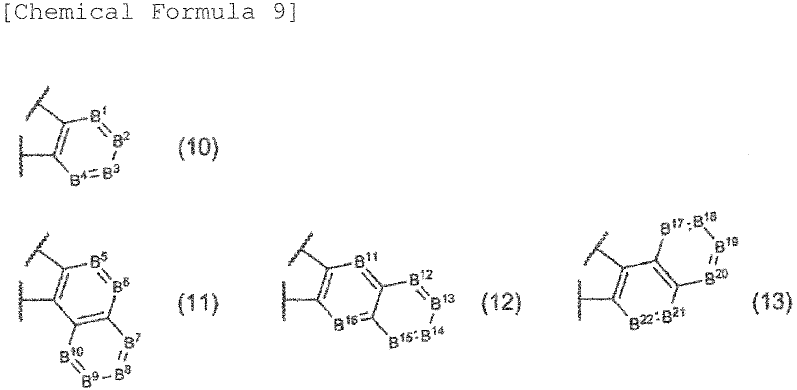 wherein B¹ to B²² represent C-R³² or N, with the proviso that when Z is a group represented by the general formula (5), at least one of B^{k}s (K = 1 to 22) contained in ring B is electron-accepting nitrogen; a substituent that B¹ to B²² optionally have is the same as in the general formula (2) ; and R³² is selected from the group consisting of hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, halogen, a carbonyl group, a carboxyl group, an oxycarbonyl group, a carbamoyl group and -P(=O)R²R³.

Ring B is not particularly limited, but is more preferably a structure represented by any of the general formulae (11) to (13). When ring B is a structure represented by any of the general formulae (11) to (13), conjugation is further expanded, so that high carrier mobility and high electron-accepting property are achieved. As a result, further low-voltage driving of the light-emitting device becomes possible, so that luminous efficiency can be further improved.

Preferably, Z is coupled to L at the position of any of ring A and ring B when Z is represented by the general formula (2); at the position of any of R¹⁶ to R¹⁹ and ring B when Z is represented by the general formula (5) ; at the position of any of R²⁰ to R²² and ring B when Z is represented by the general formula (6) ; at the position of any of R²³ to R²⁵ and ring B when Z is represented by the general formula (7); at the position of any of R²⁶ to R²⁸ and ring B when Z is represented by the general formula (8); and at the position of any of R²⁹ to R³¹ and ring B when Z is represented by the general formula (9) as described above. By coupling the group to L, the electron affinity of the fluoranthene skeleton can be properly adjusted. As a result, ease of injecting electrons into the emissive layer is further improved, and therefore the light-emitting device can be driven at a lower voltage.

The group represented by Z is not particularly limited, and specific examples thereof include groups of the following general formulae. Here, R¹ is the same as in the general formula (2) .

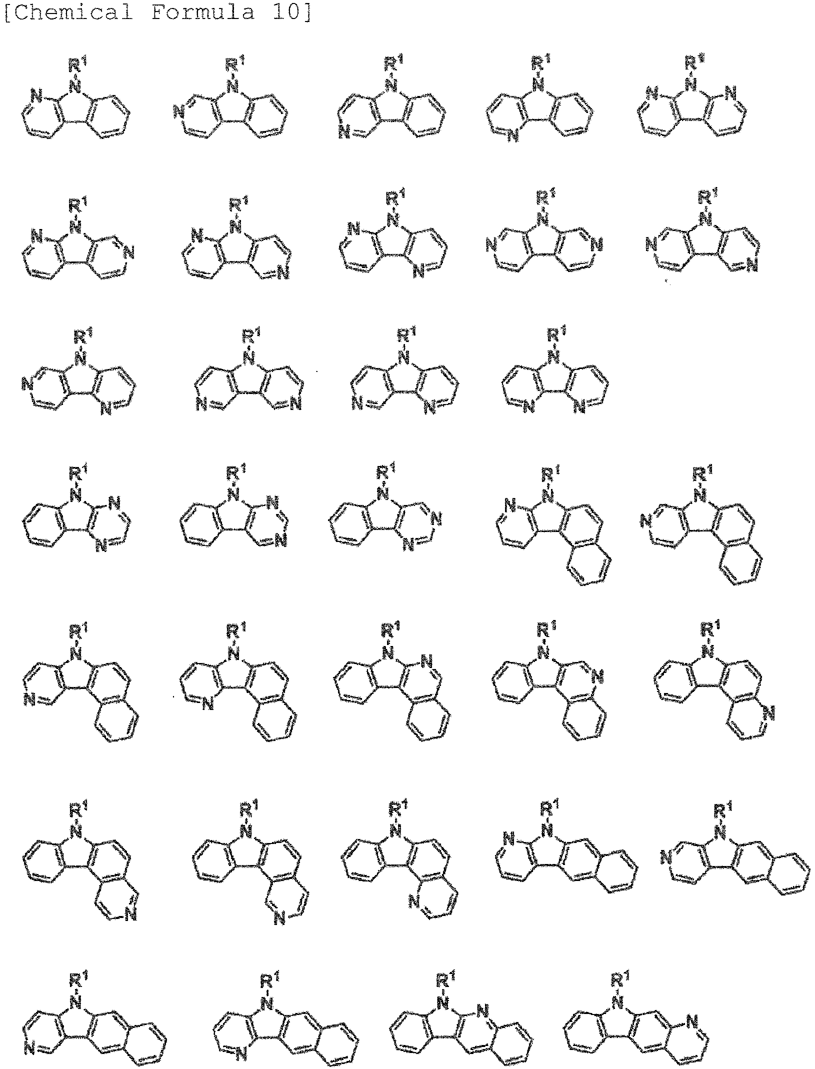

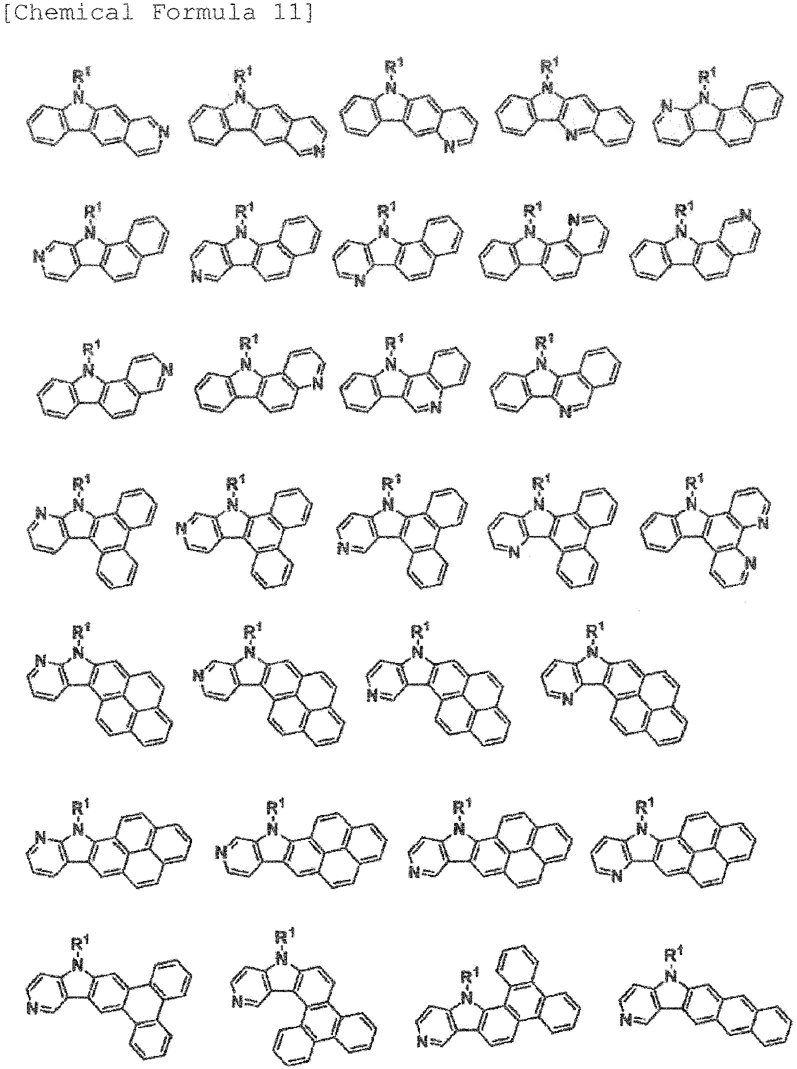

The light-emitting device material of the present invention is not particularly limited, and specific examples thereof include the following.

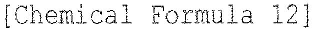

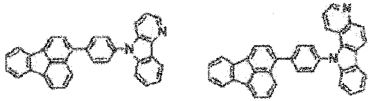

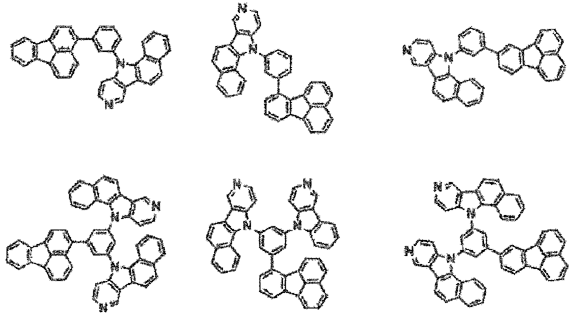

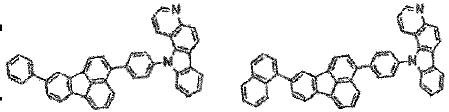

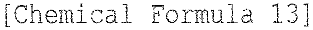

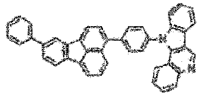

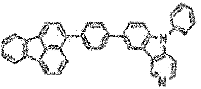

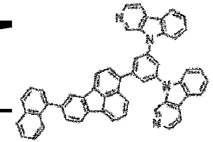

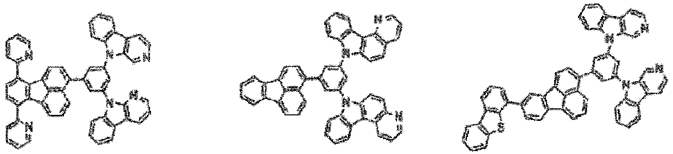

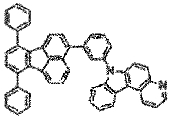

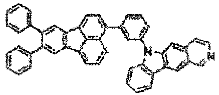

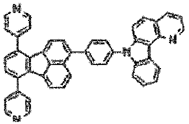

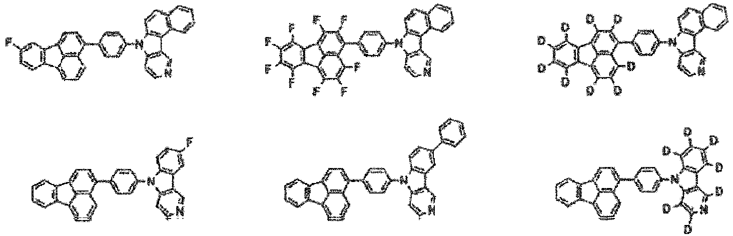

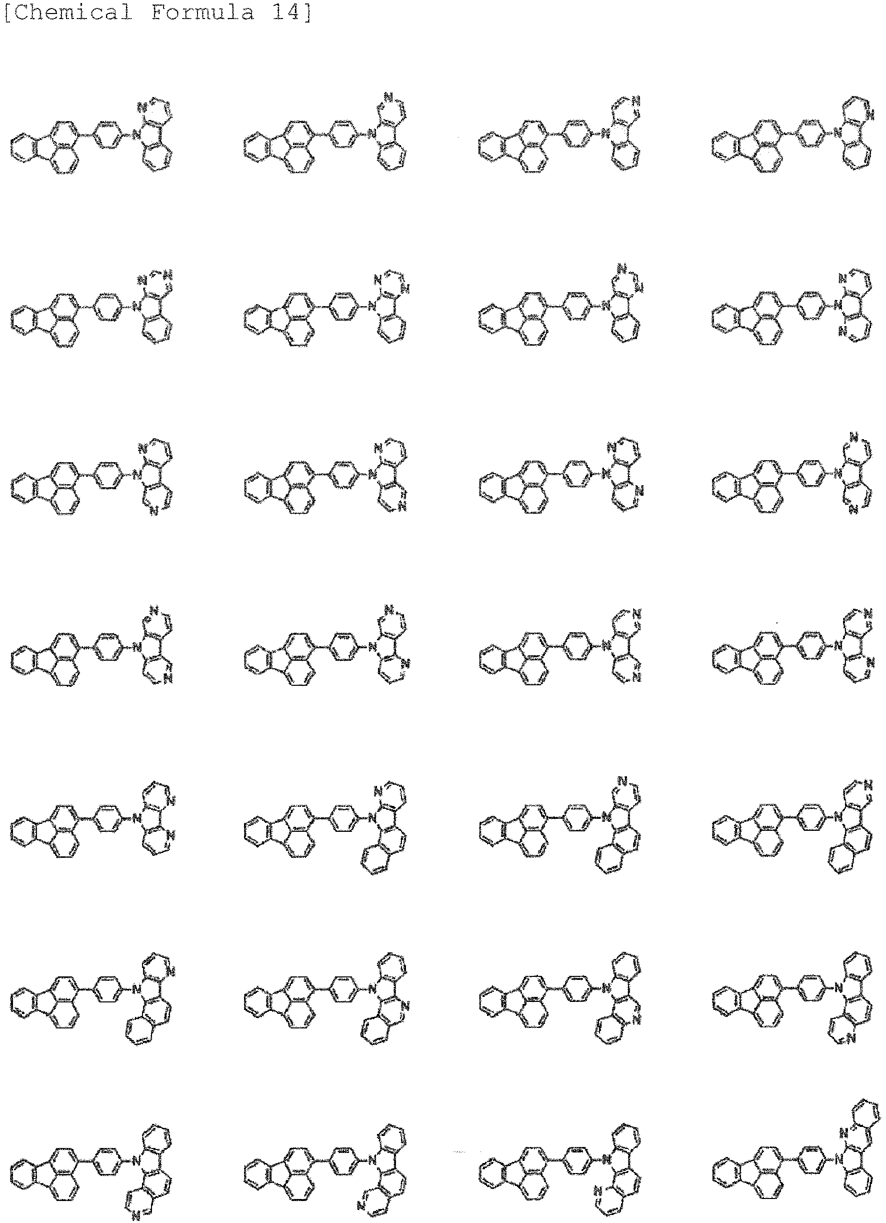

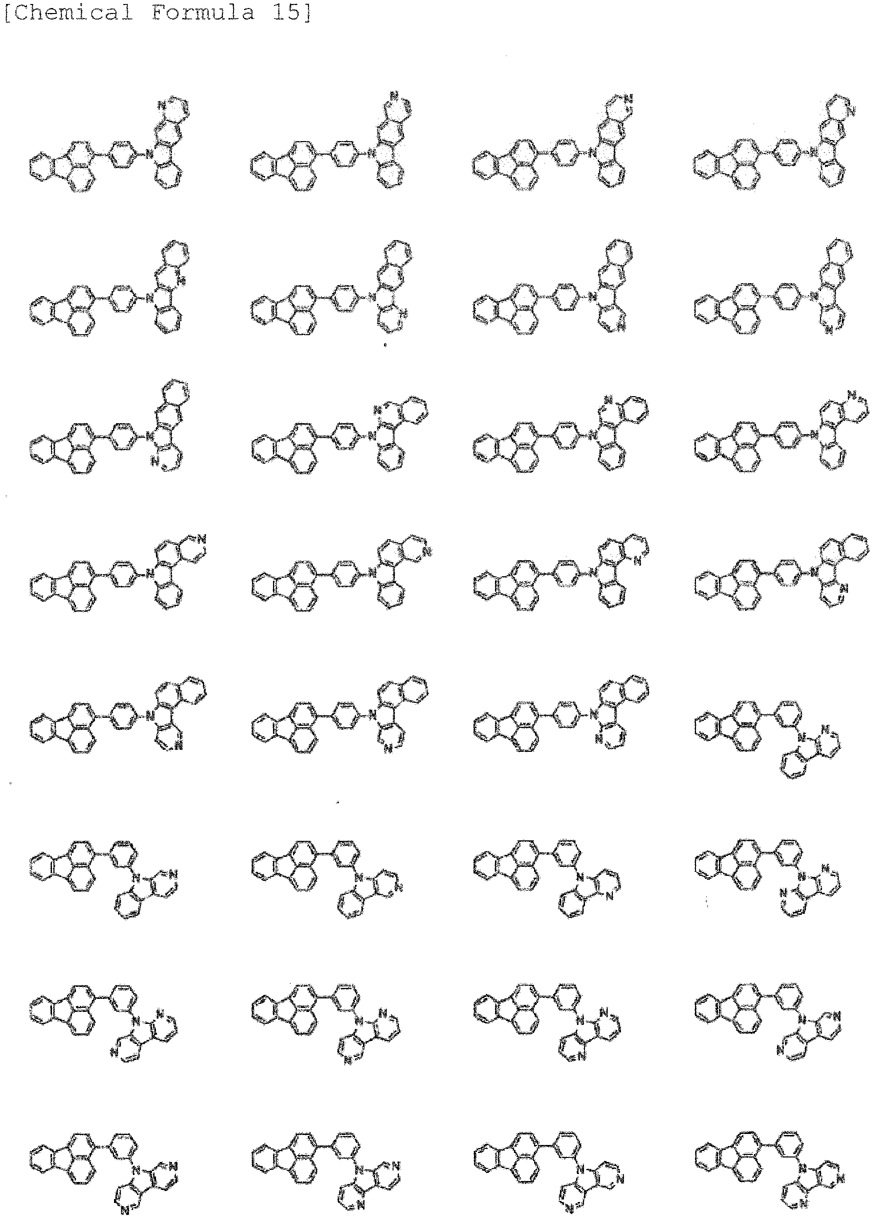

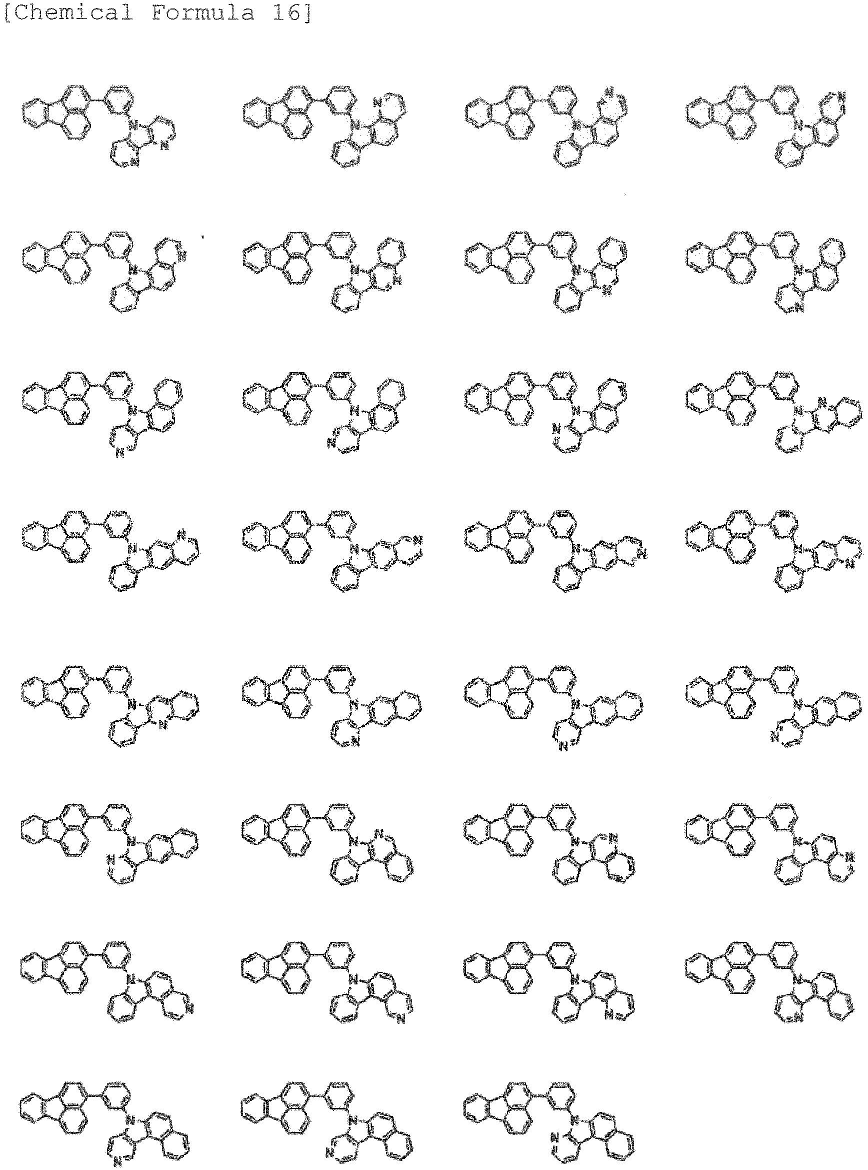

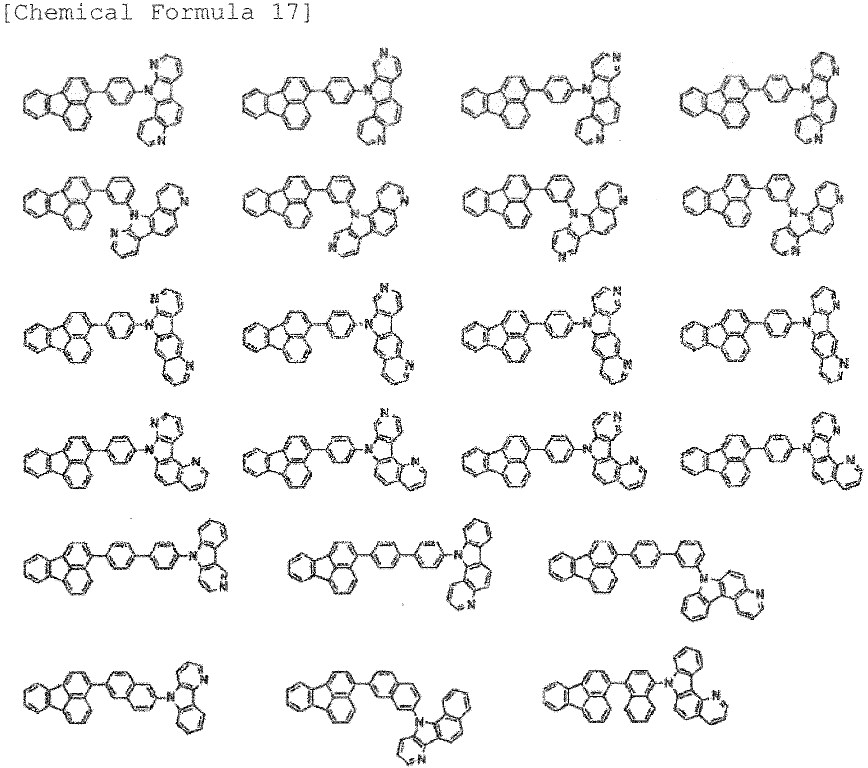

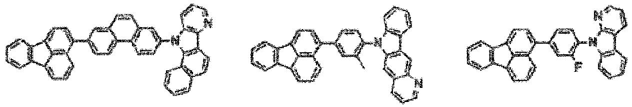

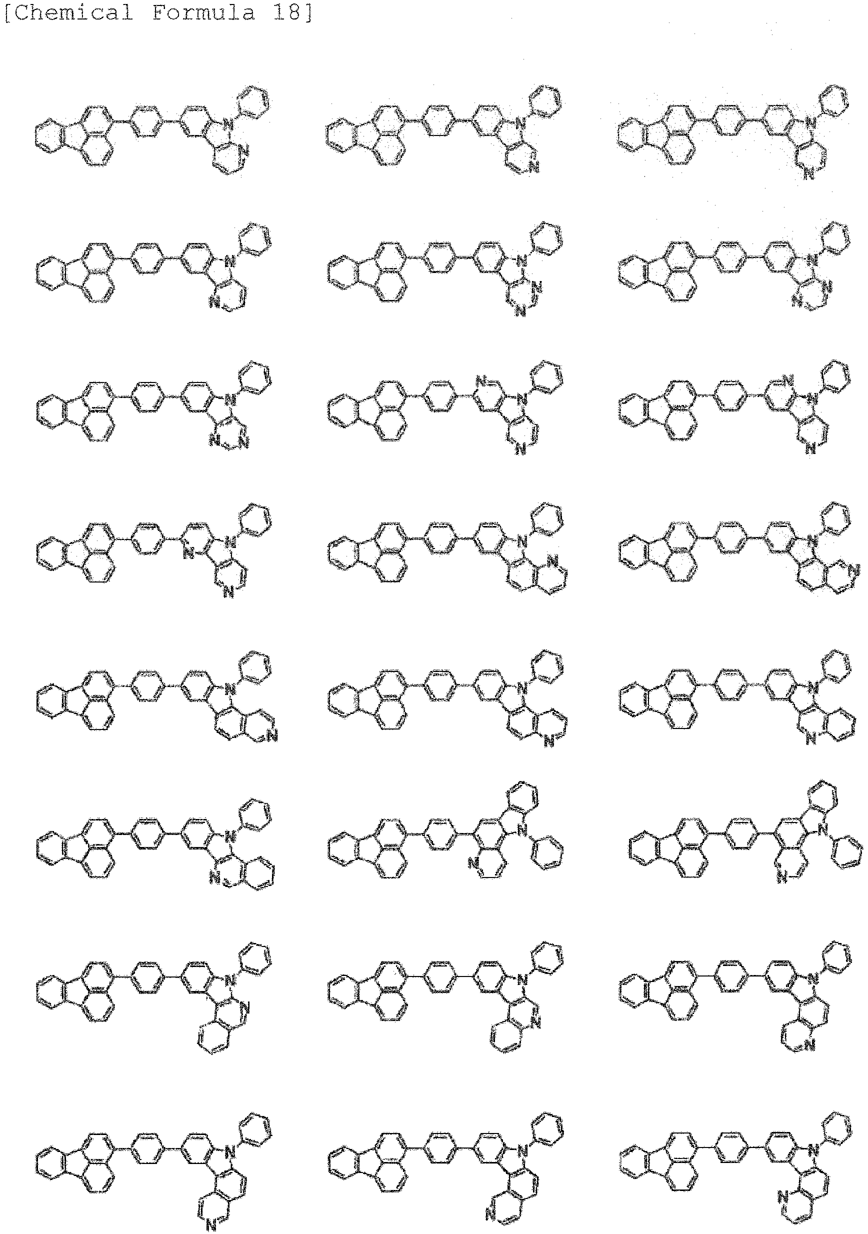

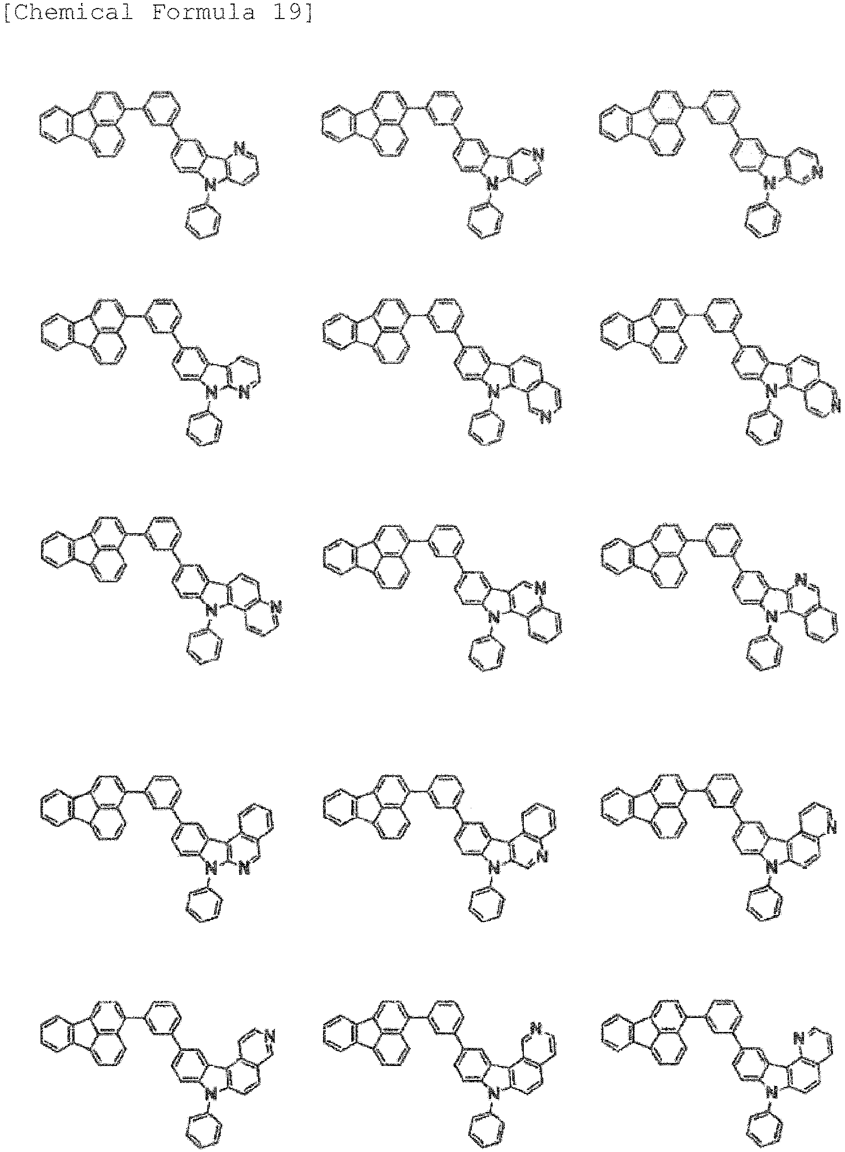

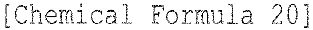

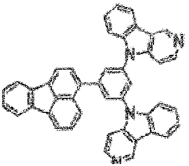

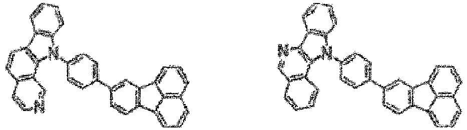

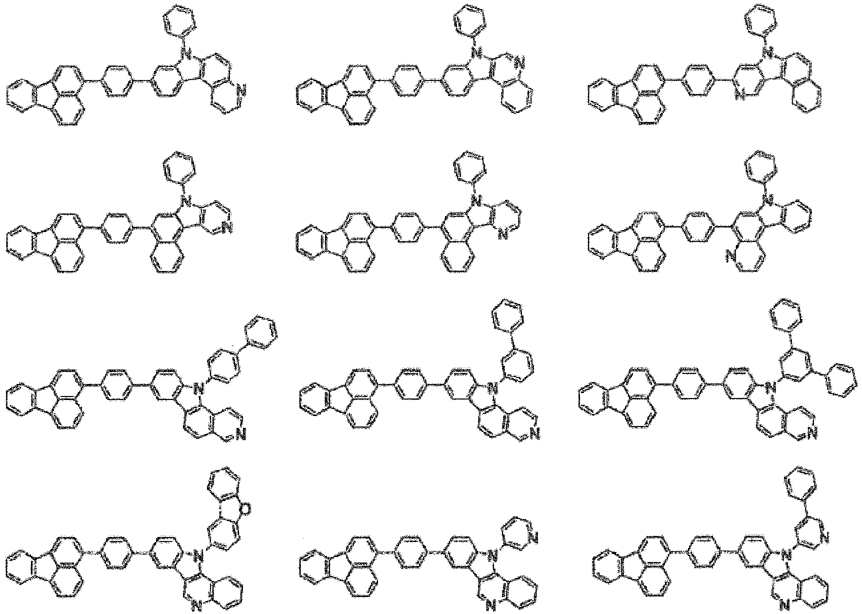

A known method can be used for synthesis of the light-emitting device material of the present invention. Examples of the method for introducing Z into the fluoranthene skeleton include, but are not limited to, a method using a coupling reaction of a substituted or unsubstituted halogenated fluoranthene derivative and substituted or unsubstituted Z under a palladium catalyst or a nickel catalyst. When Z is introduced into the fluoranthene skeleton via an arylene group or a heteroarylene group, an arylboronic acid or heteroarylboronic acid substituted with Z may be used. A boronic acid ester may be used in place of the above-mentioned various boronic acids.

The light-emitting device material of the present invention denotes a material to be used in any layer of a light-emitting device and also includes a material to be used in a protective film of a cathode, in addition to materials to be used in a hole transporting layer, an emissive layer and an electron transporting layer as described later. Use of the light-emitting device material of the present invention in any layer of a light-emitting device can afford high luminous efficiency and also can afford a light-emitting device having a low driving voltage and high durability.

Next, embodiments of the light-emitting device of the present invention will be described in detail. The light-emitting device of the present invention has an anode and a cathode, and an organic layer interposed between the anode and the cathode, the organic layer includes at least an emissive layer and an electron transporting layer, and the emissive layer emits light by electric energy.

Examples of the laminated configuration of the organic layer include, besides a configuration made up of only emissive layer/electron transporting layer, laminated configurations such as 1) hole transporting layer/emissive layer/electron transporting layer, 2) hole transporting layer/emissive layer/electron transporting layer/electron injection layer, and 3) hole injection layer/hole transporting layer/emissive layer/electron transporting layer/electron injection layer. Each of the layers may be in the form of a single layer or a plurality of layers.

The light-emitting device material of the present invention may be used for any layer in the above-mentioned device configuration, but is preferably used for the emissive layer or electron transporting layer of the light-emitting device because it has high electron injection/transporting abilities, a high fluorescence quantum yield and high thin-film stability. Particularly, the light-emitting device material has excellent electron injection/transporting abilities, and is therefore preferably used for the electron transporting layer.

In the light-emitting device of the present invention, the anode and the cathode have a role for supplying a sufficient current for light emission of the device, and it is preferred that at least one of them is transparent or translucent in order to take out light. Usually, the anode formed on a substrate is made to be a transparent electrode.

While the material to be used for the anode is not particularly limited and may be electroconductive metal oxides, such as tin oxide, indium oxide, tin oxide indium (ITO), and zinc-oxide indium (IZO); metals, such as gold, silver, and chromium; inorganic electroconductive substances, such as copper iodide and copper sulfide; or electroconductive polymers, such as polythiophene, polypyrrole, and polyaniline as long as being a material that is capable of injecting holes into an organic layer efficiently and that is transparent or translucent in order to take out light, use of ITO glass or NESA glass is particularly preferable. These electrode materials may be used alone, or a plurality of materials may be used in lamination or in admixture. Since it is favorable that a sufficient current for light emission of the device can be supplied, the resistance of a transparent electrode is not limited, but from the viewpoint of the power consumption of the device, a low resistance is desirable. For example, an ITO substrate having a resistance of 300 Ω/□ or lower functions as a device electrode, but since it is currently possible to supply a substrate having a resistance of about 10 Ω/□, it is particularly preferable to use a substrate having a low resistance of 20 Ω/□ or lower. The thickness of ITO can be arbitrarily selected according to a resistance value, but ITO is usually used at a thickness of between 100 to 300 nm in many cases.

In addition, in order to retain the mechanical strength of the light-emitting device, it is preferred to form the light-emitting device on a substrate. As the substrate, a glass substrate such as soda glass or alkali-free glass is suitably used. Since it is favorable that the thickness of a glass substrate has a sufficient thickness for retaining the mechanical strength, a thickness of 0.5 mm or more is sufficient. Regarding the material of glass, since it is preferred that the amount of ions eluted from glass is small, alkali-free glass is more preferable. Alternatively, since soda lime glass provided with a barrier coating such as SiO₂ is commercially available, it can also be used. Further, as far as the first electrode stably functions, it is not necessary that the substrate is glass and, for example, the anode may be formed on a plastic substrate. Examples of a method of forming an ITO film include, but are not particularly limited to, an electron beam method, a sputtering method, and a chemical reaction method.

A material to be used in the cathode is not particularly limited, as far as it is a substance which can efficiently inject electrons into the emissive layer. Generally, metals such as platinum, gold, silver, copper, iron, tin, aluminum, and indium, or alloys or multilayer lamination of these metals with metals having a low work function such as lithium, sodium, potassium, calcium and magnesium are preferred. Among them, as a main component, aluminum, silver, and magnesium are preferred from the viewpoints of electric resistance value, easiness of making a film, stability of a film, and luminous efficiency. In particular, it is preferred that the material is constituted by magnesium and silver because electron injection into the electron transporting layer and the electron injection layer in the present invention becomes easy, and low voltage driving becomes possible.

Further, preferable examples include lamination of metals such as platinum, gold, silver, copper, iron, tin, aluminum, and indium, or alloys using these metals, inorganic substances such as silica, titania, and silicon nitride, and organic polymer compounds such as polyvinyl alcohol, polyvinyl chloride, and a hydrocarbon-based polymer compound as a protective film layer on the cathode for protecting the cathode. The light-emitting device material of the present invention can also be used as the protective film layer. However, in the case of a device structure for taking out light from the cathode side (top emission structure), the protective film layer is selected from materials having light permeability in a visible light region. Examples of a method for preparation of these electrodes include, but are not particularly limited to, resistance heating, electron beam, sputtering, ion plating and coating.

The hole transporting layer is formed by a method in which one or more hole transporting materials are laminated or mixed, or a method using a mixture of a hole transporting material and a polymer binder. The hole transporting material is required to efficiently transport holes from a positive electrode between electrodes given an electric field, and preferably has high hole injection efficiency and efficiently transports injected holes. For this purpose, the hole transporting material is required to be a substance having an appropriate ionization potential and, moreover, great hole mobility and, further, excellent stability, and generating impurities that become a trap with difficulty at the time of production and at the time of use. The substance satisfying the above-mentioned requirements is not particularly limited, and, for example, benzidine derivatives such as 4,4'-bis(N-(3-methylphenyl)-N-phenyiamino)biphenyl (TPD), 4,4'-bis(N-(-naphthyl)-N-phenylamino)biphenyl (NPD), 4,4'-bis(N,N-bis(4-biphenylyl)amino)biphenyl (TBDB) and bis(N,N'-diphenyl-4-aminophenyl)-N,N-diphenyl-4,4'-diamino-1,1'-biphenyl (TPD232); materials called starburst arylamines, such as 4,4',4"-tris(3-methylphenyl(phenyl)amino)triphenylamine (m-MTDATA) and 4,4',4"-tris(1-naphthyl(phenyl)amino)triphenylamine (1-TNATA) ; materials having a carbazole skeleton, particularly carbazole polymers, specifically derivatives of a carbazole dimer such as bis(N-arylcarbazole) or bis(N-alkylcarbazole), derivatives of a carbazole trimer and derivatives of a carbazole tetramer; triphenylene compounds; pyrazoline derivatives; stilbene-based compounds; hydrazone-based compounds; benzofuran derivatives; heterocyclic compounds such as thiophene derivatives, oxadiazole derivatives, phthalocyanine derivatives and porphyrin derivatives; fullerene derivatives; and such polymers as polycarbonates and styrene derivatives having the aforementioned monomers on their side chains, polythiophene, polyaniline, polyfluorene, polyvinylcarbazole and polysilane are preferred. Further, inorganic compounds such as p-type Si and p-type SiC can also be used.

The light-emitting device material of the present invention can also be used as a hole transporting material because it has great hole mobility and, further, excellent electrochemical stability. The light-emitting device material of the present invention may be used as a hole injection material, but is suitably used as a hole transporting material because it has high hole mobility.

The light-emitting device material of the present invention has excellent electron injection/transporting properties, and therefore when the light-emitting device material is used for the electron transporting layer, there is the possibility that electrons are not recombined in the emissive layer, and are partially leaked to the hole transporting layer. Therefore, it is preferred that a compound excellent in electron blocking property is used for the hole transporting layer. Particularly, a compound containing a carbazole skeleton is preferred because it is excellent in electron blocking property, and can contribute to an increase in efficiency of the light-emitting device. Further, it is preferred that the compound containing a carbazole skeleton contains a carbazole dimer, carbazole trimer or carbazole tetramer skeleton. This is because they have both proper electron blocking property and proper hole injection/transporting properties. Further, when the compound containing a carbazole skeleton is used for the hole transporting layer, it is more preferable that an emissive layer to be combined contains the later-described phosphorescence emitting material. This is because the compound having a carbazole skeleton has a high triplet exciton blocking function, so that luminous efficiency can be increased when the compound is combined with a phosphorescence emitting material. Use of a triphenylene skeleton-containing compound excellent in having high hole mobility for the hole transporting layer is preferred because a carrier balance is improved, so that the effects of improving luminous efficiency and improving durable life can be obtained. It is further preferable that the compound containing a triphenylene skeleton has two or more diarylamino groups. The compound containing a carbazole skeleton and the compound containing a triphenylene skeleton may be each used alone as a hole transporting layer, or may be mixed and used. Other materials may be mixed as long as the effects of the present invention are not impaired. When the hole transporting layer includes a plurality of layers, any one layer should contain the compound containing a carbazole skeleton or the compound containing a triphenylene skeleton.

A hole injection layer may be provided between the anode and the hole transporting layer. When a hole injection layer is provided, the light-emitting device has a reduced driving voltage, and durable life is improved. A material having an ionization potential smaller than that of a material which is usually used for the hole transporting layer is used for the hole injection layer. Specific examples of the material include benzidine derivatives such as TPD232, and starburst arylamine materials, and besides, phthalocyanine derivatives can also be used. It is preferred that the hole injection layer is formed of an acceptor compound alone, or the hole injection layer is used with another hole transporting material doped with an acceptor compound. Examples of the acceptor compound include metal chlorides such as iron(III) chloride, aluminum chloride, gallium chloride, indium chloride, and antimony chloride; metal oxides such as molybdenum oxide, vanadium oxide, tungsten oxide, and ruthenium oxide; and charge transfer complexes such as tris(4-bromophenyl)aminium hexachloroantimonate (TBPAH). Moreover, organic compounds having a nitro group, a cyano group, halogen, or a trifluoromethyl group in the molecule, quinone-based compounds, acid anhydride-based compounds, and fullerene can also be used suitably. Specific examples of such compounds include hexacyanobutadiene, hexacyanobenzene, tetracyanoethylene, tetracyanoquinodimethane (TCNQ), tetrafluorotetracyanoquinodimethane (F4-TCNQ), 2,3,6,7,10,11-hexacyano-1,4,5,8,9,12-hexaazatriphenylene (HAT-CN6), p-fluoranil, p-chloranil, p-bromanil, p-benzoquinone, 2,6-dichlorobenzoquinone, 2,5-dichlorobenzoquinone, tetramethylbenzoquinone, 1,2,4,5-tetracyanobenzene, o-dicyanobenzene, p-dicyanobenzene, 1,4-dicyanotetrafluorobenzene, 2,3-dichloro-5,6-dicyanobenzoquinone, p-dinitrobenzene, m-dinitrobenzene, o-dinitrobenzene, p-cyanonitrobenzene, m-cyanonitrobenzene, o-cyanonitrobenzene, 1,4-naphthoquinone, 2,3-dichloronaphthoquinone, 1-nitronaphthalene, 2-nitronaphthalene, 1,3-dinitronaphthalene, 1,5-dinitronaphthalene, 9-cyanoanthracene, 9-nitroanthracene, 9,10-anthraquinone, 1,3,6,8-tetranitrocarbazole, 2,4,7-trinitro-9-fluorenone, 2,3,5,6-tetracyanopyridine, maleic anhydride, phthalic anhydride, C60, and C70.

Of these, metal oxides and cyano group-containing compounds are preferred because they can be easily handled and deposited, and therefore the above-described effects can be obtained easily. Examples of the preferred metal oxide include molybdenum oxide, vanadium oxide and ruthenium oxide. Among cyano group-containing compounds, (a) a compound having in the molecule at least one electron-accepting nitrogen atom in addition to the nitrogen atom of the cyano group, (b) a compound having in the molecule both halogen and a cyano group, (c) a compound having in the molecule both a carbonyl group and a cyano group, or (d) a compound having in the molecule both halogen and a cyano group and further, at least one electron-accepting nitrogen in addition to the nitrogen atom of the cyano group is more preferable because it serves as a strong electron acceptor. Specific examples of the above-mentioned compound include the following compounds.

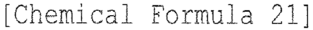

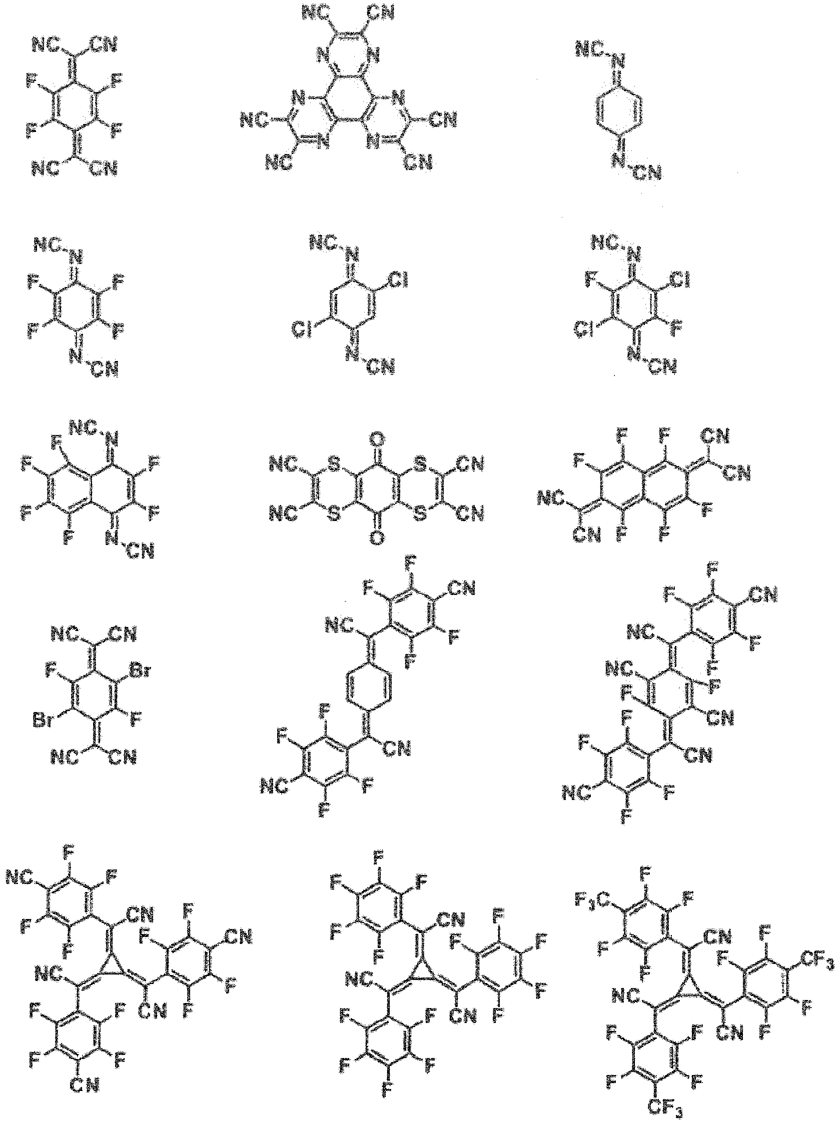

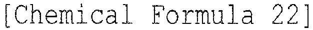

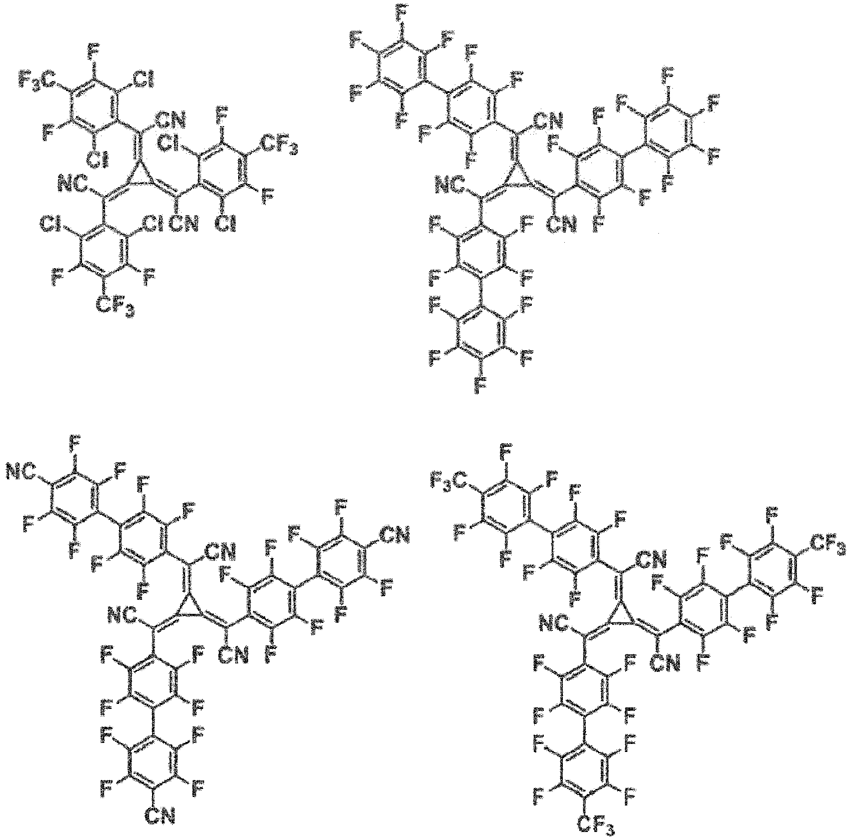

In either of the case where a hole injection layer is formed of an acceptor compound alone or the case where a hole injection layer is doped with an acceptor compound, the hole injection layer may be a single layer or may be a laminate of a plurality of layers. The hole injection material to be used in combination when the hole injection layer is doped with an acceptor compound is preferably the same compound as the compound to be used for the hole transporting layer because a barrier to injection of holes into the hole transporting layer can be mitigated.

The emissive layers may be in the form of a single layer or a plurality of layers, each of which is formed of an emissive material (host material, dopant material), and this may be a mixture of the host material and the dopant material, or the host material alone. That is, in the light-emitting device of the present invention, only the host material or the dopant material may emit light, or both of the host material and the dopant material may emit light, in each emissive layer. From the viewpoints that electric energy is efficiently utilized and light emission at high color purity is obtained, it is preferred that the emissive layer includes a mixture of the host material and the dopant material. In addition, the host material and the dopant material may be one kind or a combination of a plurality of kinds, respectively. The dopant material may be contained in a whole host material, or may be partially contained therein. The dopant material may be laminated, or may be dispersed. The dopant material can control an emitted color. When the amount of the dopant material is too large, concentration quenching occurs, and therefore the dopant material is preferably used in an amount of 20% by weight or less, further preferably 10% by weight or less based on the host material. As a doping method, the dopant material can be co-deposited with the host material, or the dopant material may be mixed with the host material in advance to be deposited simultaneously.

Specific examples of the emissive material that can be used include, but are not particularly limited to, fused ring derivatives such as anthracene and pyrene, metal chelated oxinoid compounds including tris(8-quinolinolato)aluminum, bisstyryl derivatives such as bisstyrylanthracene derivatives and distyrylbenzene derivatives, tetraphenylbutadiene derivatives, indene derivatives, coumarin derivatives, oxadiazole derivatives, pyrrolopyridine derivatives, perinone derivatives, cyclopentadiene derivatives, oxadiazole derivatives, thiadiazolopyridine derivatives, dibenzofuran derivatives, carbazole derivatives, and indolocarbazole derivatives and, as a polymer series, polyphenylenevinylene derivatives, polyparaphenylene derivatives, and polythiophene derivatives, which have hitherto been known as a light emitting body.

The host material contained in the emissive material is not particularly limited, and examples of the host material which can be used include, but are not particularly limited to, compounds having a fused aryl ring such as naphthalene, anthracene, phenanthrene, pyrene, chrysene, naphthacene, triphenylene, perylene, fluoranthene, fluorene and indene, and derivatives thereof, aromatic amine derivatives such as N,N'-dinaphthyl-N,N'-diphenyl-4,4'-diphenyl-1,1'-diamine, metal chelated oxinoid compounds including tris(8-quinolinato)aluminum (III), bisstyryl derivatives such as distyrylbenzene derivatives, tetraphenylbutadiene derivatives, indene derivatives, coumarin derivatives, oxadiazole derivatives, pyrrolopyridine derivatives, perinone derivatives, cyclopentadiene derivatives, pyrrolopyrrole derivatives, thiadiazolopyridine derivatives, dibenzofuran derivatives, carbazole derivatives, indolocarbazole derivatives and triazine derivatives and, as a polymer series, polyphenylenevinylene derivatives, polyparaphenylene derivatives, polyfluorene derivatives, polyvinylcarbazole derivatives, and polythiophene derivatives. The dopant material is not particularly limited, and examples of the dopant material that can be used include compounds having a fused aryl ring such as naphthalene, anthracene, phenanthrene, pyrene, chrysene, triphenylene, perylene, fluoranthene, fluorene and indene, and derivatives thereof (e.g., 2-(benzothiazol-2-yl)-9,10-diphenylanthracene and 5,6,11,12-tetraphenylnaphthacene); compounds having a heteroaryl ring such as furan, pyrrole, thiophene, silole, 9-silafluorene, 9,9'-spirobisilafluorene, benzothiophene, benzofuran, indole, dibenzothiophene, dibenzofuran, imidazopyridine, phenanthroline, pyridine, pyrazine, naphthyridine, quinoxaline, pyrrolopyridine and thioxanthene, and derivatives thereof; borane derivatives; distyrylbenzene derivatives; aminostyryl derivatives such as 4,4'-bis(2-(4-diphenylaminophenyl)ethenyl)biphenyl and 4,4'-bis(N-(stilben-4-yl)-N-phenylamino)stilbene; aromatic acetylene derivatives; tetraphenylbutadiene derivatives; stilbene derivatives; aldazine derivatives; pyrromethene derivatives; diketopyrrolo[3,4-c]pyrrole derivatives; coumarin derivatives such as 2,3,5,6-1H,4H-tetrahydro-9-(2'-benzothiazolyl)quinolizino[9 ,9a,1-gh]coumarin; azole derivatives such as imidazole, thiazole, thiadiazole, carbazole, oxazole, oxadiazole and triazole, and metal complexes thereof; and aromatic amine derivatives typified by N,N'-diphenyl-N,N'-di(3-methylphenyl)-4,4'-diphenyl-1,1'-di amine.

The emissive layer may contain a phosphorescence emitting material. The phosphorescence emitting material is a material that emits phosphorescence at room temperature. When a phosphorescence emitting material is used as a dopant, basically it is required to obtain phosphorescence emission at room temperature, but the phosphorescence emitting material is not particularly limited, and is preferably an organic metal complex compound containing at least one metal selected from the group consisting of iridium (Ir), ruthenium (Ru), rhodium (Rh), palladium (Pd), platinum (Pt), osmium (Os), and rhenium (Re). Among them, an organic metal complex having iridium or platinum is preferred because it has a high phosphorescence emission yield at room temperature. As the host to be used in combination with a phosphorescence emitting dopant, aromatic hydrocarbon compound derivatives such as indole derivatives, carbazole derivatives, indolocarbazole derivatives, nitrogen-containing aromatic compound derivatives having a pyridine, pyrimidine or triazine skeleton, polyarylbenzene derivatives, spirofluorene derivatives, truxene derivatives and triphenylene derivatives; compounds containing a chalcogen element, such as dibenzofuran derivatives and dibenzothiophene derivatives; organic metal complexes such as beryllium quinolinol complexes; and the like are suitably used, but the host is not limited thereto as long as basically it has higher triplet energy than a dopant used, and electrons and holes are smoothly injected into and transported from the respective transporting layers. Two or more triplet emissive dopants may be contained, and two or more host materials may be contained. Further, one or more triplet emissive dopants and one or more fluorescence emitting dopants may be contained.

The preferable phosphorescence emitting host or dopant is not particularly limited, and specific examples thereof include the following.

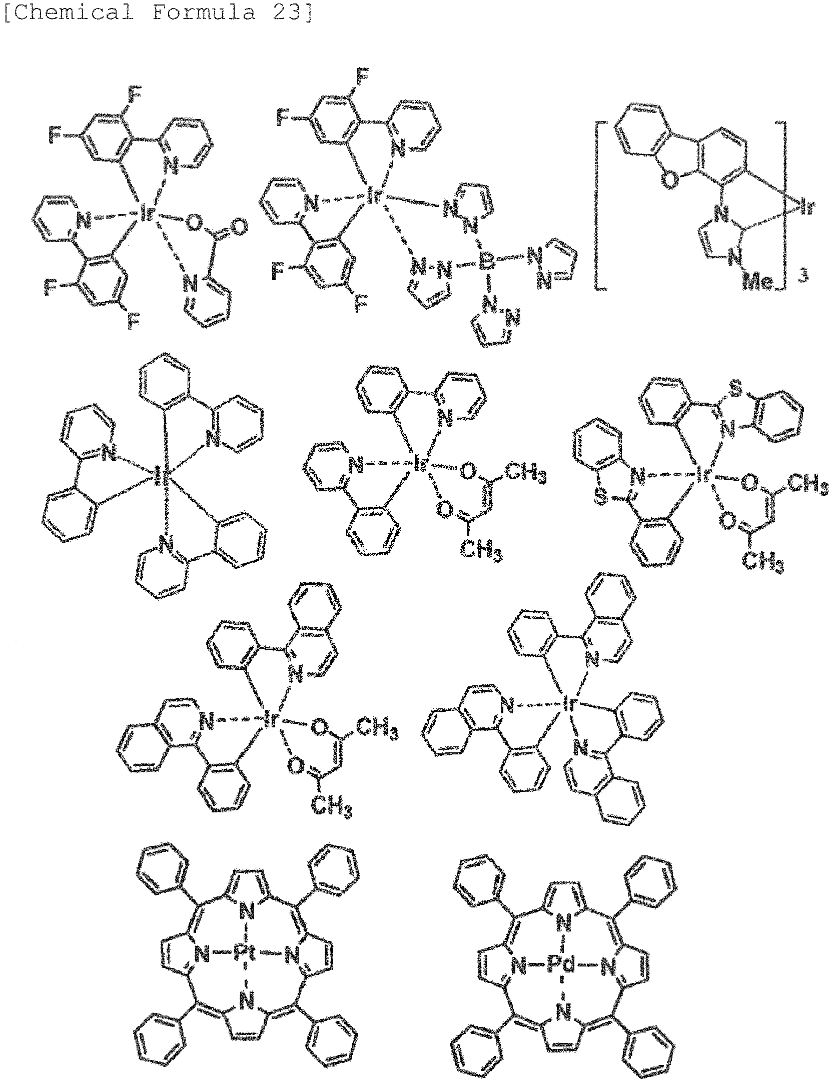

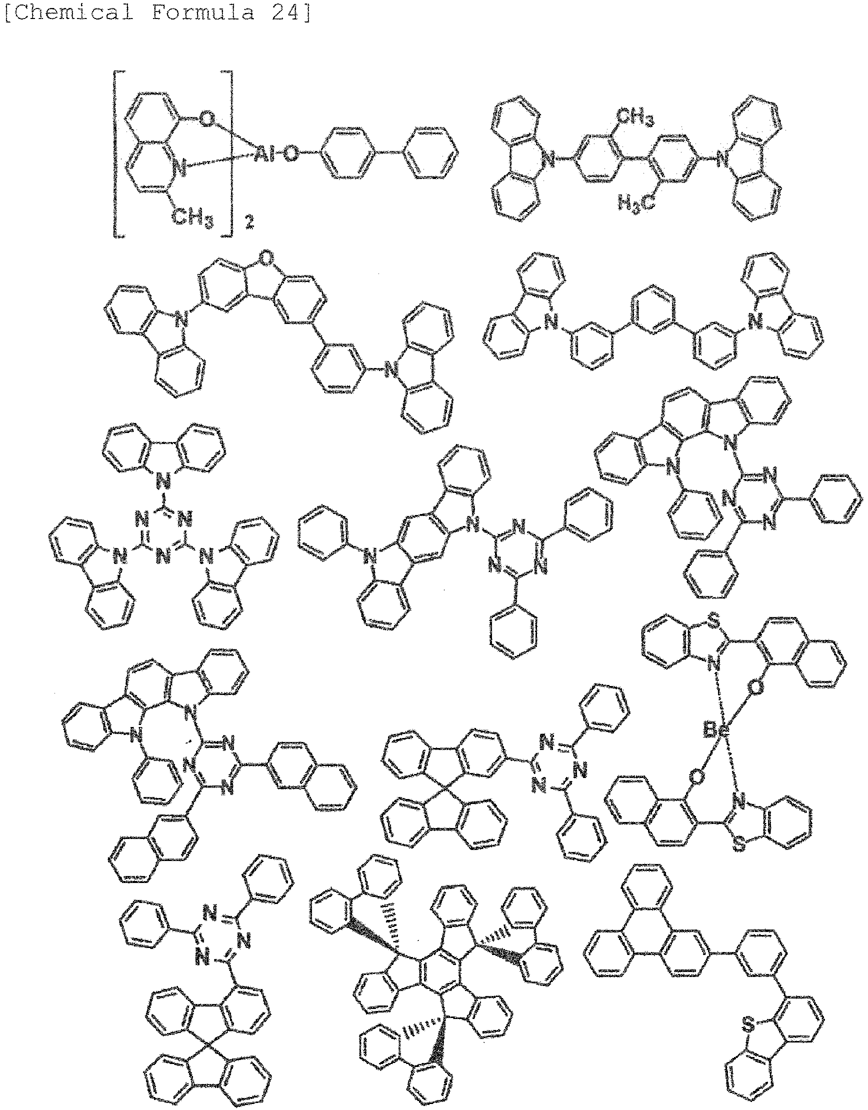

The light-emitting device material of the present invention can also be used as an emissive material because it has high light emitting performance. The light-emitting device material of the present invention can be suitably used as a blue light emitting material because it shows intensive luminescence in an ultraviolet to blue region (300 to 500 nm region). The light-emitting device material of the present invention may be used as a host material, but is suitably used as a dopant material because it has a high fluorescence quantum yield.

In the present invention, the electron transporting layer is a layer in which electrons are injected from the cathode and, further, which transports the electrons. It is desired that the electron transporting layer has a high electron injection efficiency, and efficiently transports injected electrons. For this purpose, it is preferred that the electron transporting layer is formed of a substance having great electron affinity and, moreover, great electron mobility and, further, excellent stability, and generating impurities that become a trap with difficulty at the time of production and at the time of use. However, when transportation balance between holes and electrons is considered, if the electron transporting layer mainly plays a role of being able to efficiently inhibiting holes from the anode from flowing to the cathode side without recombination, even when the layer is constituted by a material having not so high electron transporting ability, the effect of improving luminous efficiency becomes equivalent to that when the layer is constituted by a material having a high electron transporting ability. Therefore, the electron transporting layer in the present invention also includes a hole inhibition layer which can efficiently inhibit the transfer of holes as the same meaning.

Examples of the electron transporting material to be used for the electron transporting layer include fused polycyclic aromatic derivatives, such as naphthalene and anthracene; styryl-based aromatic ring derivatives typified by 4,4'-bis(diphenylethenyl)biphenyl; quinone derivatives, such as anthraquinone and diphenoquinone; phosphorus oxide derivatives; and various types of metal complexes, such as quinolinol complexes, e.g., tris(8-quinolinolato)aluminum(III), benzoquinolinol complexes, hydroxyazole complexes, azomethine complexes, tropolone metal complexes, and flavonol metal complexes. It is preferred to use a compound that includes an element selected from carbon, hydrogen, nitrogen, oxygen, silicon and phosphorus, and has a heteroaryl ring structure containing electron-accepting nitrogen because it can reduce a driving voltage and highly efficient light emission can be obtained.

An aromatic heterocyclic ring containing electron-accepting nitrogen has high electron affinity. An electron transporting material having electron-accepting nitrogen makes easier acceptance of electrons from a cathode having higher electron affinity, and lower voltage driving becomes possible. In addition, since supply of electrons to an emissive layer is increased and a recombining probability is increased, luminous efficiency is improved.

Examples of the heteroaryl ring containing electron-accepting nitrogen include a pyridine ring, a pyrazine ring, a pyrimidine ring, a quinoline ring, a quinoxaline ring, a naphthyridine ring, a pyrimidopyrimidine ring, a benzoquinoline ring, a phenanthroline ring, an imidazole ring, an oxazole ring, an oxadiazole ring, a triazole ring, a thiazole ring, a thiadiazole ring, a benzoxazole ring, a benzothiazole ring, a benzimidazole ring, and a phenanthroimidazole ring.

Examples of preferred compounds having such a heteroaryl ring structure include benzimidazole derivatives, benzoxazole derivatives, benzothiazole derivatives, oxadiazole derivatives, thiadiazole derivatives, triazole derivatives, pyrazine derivatives, phenanthroline derivatives, quinoxaline derivatives, quinoline derivatives, benzoquinoline derivatives, oligopyridine derivatives such as bipyridine and terpyridine, quinoxaline derivatives and naphthyridine derivatives. Among them, imidazole derivatives such as tris(N-phenylbenzimidazol-2-yl)benzene; oxadiazole derivatives such as 1,3-bis[(4-tert-butylphenyl)1,3,4-oxadiazolyl]phenylene; triazole derivatives such as N-naphthyl-2,5--diphenyl-1,3,4-triazole; phenanthroline derivatives such as bathocuproine and 1,3-bis(1,10-phenanthrolin-9-yl)benzene; benzoquinoline derivatives such as 2,2'-bis(benzo[h]quinolin-2-yl)-9,9'-spirobifluorene; bipyridine derivatives such as 2,5-bis(6'-(2',2"-bipyridyl))-1,1-dimethyl-3,4-diphenylsilo le; terpyridine derivatives such as 1,3-bis(4'-(2,2':6'2"-terpyridinyl))benzene; and naphthyridine derivatives such as bis(1-naphthyl)-4-(1,8-naphthyridin-2-yl)phenylphosphine oxide are suitably used in view of an electron transporting ability. It is more preferable that such a derivative has a fused polycyclic aromatic skeleton because if so, then the glass transition temperature will increase and an effect of reducing the voltage of a light-emitting device is great due to increased electron mobility. Moreover, considering the improvement in durable life of a device, the easiness of synthesis, and easy availability of raw materials, it is particularly preferable that the fused polycyclic aromatic skeleton is an anthracene skeleton, a pyrene skeleton, or a phenanthroline skeleton. While the electron transporting material may be used alone, two or more kinds of the electron transporting materials may be used in combination, or one or more kinds of other electron transporting materials may be used in a combination with the electron transporting material.

Preferable electron transporting materials are not particularly limited, and specific examples thereof include the following.

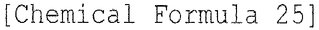

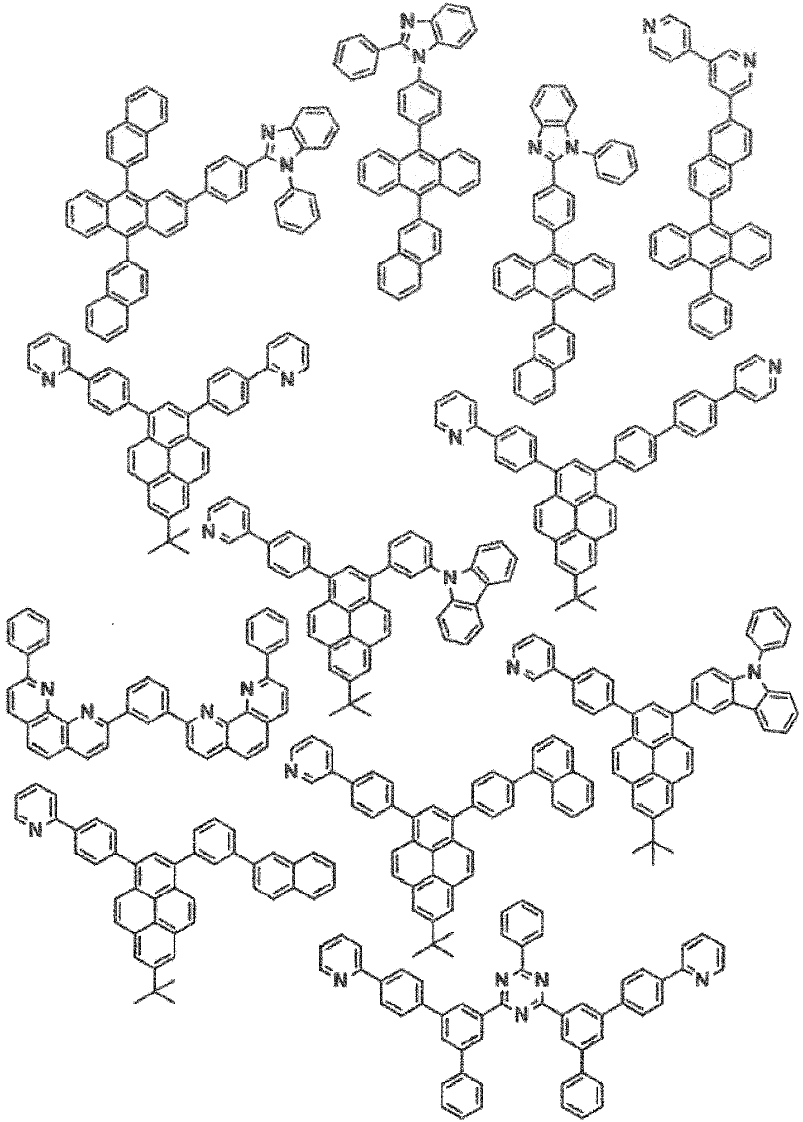

Besides these electron transporting materials, those disclosed in WO 2004-63159, WO 2003-60956, Appl. Phys. Lett. 74, 865 (1999), Org. Electron. 4, 113 (2003), WO 2010-113743 and WO 2010-1817 can be used.

The light-emitting device material of the present invention can also be used as an electron transporting material because it has high electron injection/transporting abilities.

When the light-emitting device material of the present invention is used, it does not need to be restricted to each one type, and a plurality of fluoranthene compounds according to the present invention may be used in admixture, or one or more of other electron transporting materials may be used in admixture with the fluoranthene compound according to the present invention as long as the effects of the present invention are not impaired. The electron transporting material that can be mixed is not particularly limited, and examples thereof include compounds having a fused aryl ring, such as naphthalene, anthracene and pyrene, and derivatives thereof; styryl-based aromatic ring derivatives typified by 4,4'-bis(diphenylethenyl)biphenyl; perylene derivatives; perinone derivatives; coumarin derivatives; naphthalimide derivatives; quinone derivatives such as anthraquinone and diphenoquinone; phosphorus oxide derivatives; carbazole derivatives; indole derivatives; quinolinol complexes such as tris(8-quinolinolato)aluminum(III); hydroxyazole complexes such as hydroxyphenyloxazole complexes; azomethine complexes; tropolone metal complexes; and flavonol metal complexes.

While the electron transporting material may be used alone, two or more kinds of the electron transporting materials may be used in combination, or one or more kinds of other electron transporting materials may be used in combination with the electron transporting material. Moreover, a donor material may be contained. The donor material denotes a compound which makes easy electron injection into the electron transporting layer from the cathode or the electron injection layer and, moreover, improves the electric conductivity of the electron transporting layer, by improving an electron injection barrier.

Preferable examples of the donor material in the present invention include an alkali metal, an inorganic salt containing an alkali metal, a complex of an alkali metal and an organic substance, an alkaline earth metal, an inorganic salt containing an alkaline earth metal, and a complex of an alkaline earth metal and an organic substance. Examples of the preferable kind of the alkali metal and the alkaline earth metal include alkali metals such as lithium, sodium and cesium, and alkaline earth metals such as magnesium and calcium which have a low work function and have a great effect of improving electron transporting ability.

In addition, since deposition in vacuum is easy and handling is excellent, the donor compound is preferably in the state of an inorganic salt or a complex with an organic substance rather than a metal single substance. Moreover, from the viewpoints of improvement in easiness in handling in the atmospheric air and easiness in control of the concentration to be added, the donor compound is more preferably in the state of a complex with an organic substance. Examples of the inorganic salt include oxides such as LiO and Li₂O, nitrides, fluorides such as LiF, NaF and KF, and carbonates such as Li₂CO₃, Na₂CO₃, K₂CO₃, Rb₂CO₃ and Cs₂CO₃. Preferable examples of the alkali metal or alkaline earth metal include lithium from the viewpoints of an inexpensive raw material and ease of synthesis . In addition, preferable examples of the organic substance in complexes with an organic substance include quinolinol, benzoquinolinol, flavonol, hydroxyimidazopyridine, hydroxybenzazole, and hydroxytriazole. Particularly, a complex of an alkali metal and an organic substance is preferred, a complex of lithium and an organic substance is more preferred, and lithium quinolinol is especially preferred. Two or more of these donor materials may be used in admixture.

The preferred doping concentration varies depending on a material and a film thickness of the doping region, but for example when the donor material is an inorganic material such as an alkali metal or an alkaline earth metal, it is preferred that an electron transporting layer is formed by performing co-deposition so that the deposition rate ratio of an electron transporting material and a donor material is within the range of 10000 : 1 to 2 : 1. The deposition rate ratio is more preferably 100 : 1 to 5 : 1, further preferably 100 : 1 to 10 : 1. When the donor material is a complex of a metal and an organic substance, it is preferred that an electron transporting layer is formed by performing co-deposition so that the deposition rate ratio of an electron transporting material and the donor compound is within the range of 100 : 1 to 1 : 100. The deposition rate ratio is more preferably 10 : 1 to 1 : 10, further preferably 7 : 3 to 3 : 7.

An electron transporting layer with the light-emitting device material of the present invention doped with a donor material as described above may be used as a charge generation layer in a tandem structure type device in which a plurality of light-emitting devices are coupled.

The method in which an electron transporting layer is doped with a donor material to improve an electron transporting ability exhibits an effect particularly when the film thickness of a thin-film layer is large. The method is particularly preferably used when the total film thickness of the electron transporting layer and the emissive layer is 50 nm or more. For example, there is a method in which an interference effect is used for improving luminous efficiency, and the method is intended to improve light extraction efficiency by matching the phases of light emitted directly from an emissive layer and light reflected at a cathode. The optimum conditions thereof vary depending on a light emitting wavelength, and the total film thickness of the electron transporting layer and the emissive layer becomes 50 nm or more, and may become a large film thickness close to 100 nm in the case of emission of light having a long wavelength, such as red light.

The film thickness of the electron transporting layer, which is doped, may be a part or the whole of the electron transporting layer. When a part of the electron transporting layer is doped, it is desirable to provide a doped-region at least at an electron transporting layer/cathode interface, and the effect of reducing a voltage is obtained by merely doping the vicinity of the cathode interface. On the other hand, when the donor material is in direct contact with the emissive layer, an adverse effect of reducing luminous efficiency may be caused, and in this case, it is preferred to provide a non-doped-region at an emissive layer/electron transporting layer interface.

In the present invention, an electron injection layer may be provided between a cathode and an electron transporting layer. Generally, the electron injection layer is inserted for the purpose of aiding injection of electrons from the cathode into the electron transporting layer, and when the electron injection layer is inserted, a compound having a heteroaryl ring structure containing electron-receiving nitrogen may be used, or a layer containing the above-mentioned donor material may be used. The light-emitting device material of the present invention may be contained in the electron injection layer. An inorganic substance such as an insulator or a semiconductor can also be used for the electron injection layer. Use of such a material is preferred because a short-circuit of the light-emitting device can be effectively prevented, and electron injection property can be improved. It is preferred that at least one metal compound selected from the group consisting of an alkali metal chalcogenide, an alkaline earth metal chalcogenide, a halide of an alkali metal and a halide of an alkaline earth metal is used as the insulator. It is preferred that the electron injection layer is formed of the above-mentioned alkali metal chalcogenide and the like because electron injection property can be further improved. Specifically, examples of the preferable alkali metal chalcogenide include Li₂O, Na₂S and Na₂Se, and examples of the preferable alkaline earth metal chalcogenide include CaO, BaO, SrO, BeO, BaS and CaSe. Examples of the preferable halide of an alkali metal include LiF, NaF, KF, LiCl, KCl and NaCl. Examples of the preferable halide of an alkaline earth metal include fluorides such as CaF₂, BaF₂, SrF₂, MgF₂ and BeF₂, and halides other than fluorides. Further, a complex of an organic substance and a metal is suitably used. Use of a complex of an organic substance and a metal for the electron injection layer is preferred because the film thickness is easily adjusted. As examples of the above-mentioned organic metal complex, preferable examples of the organic substance in complexes with an organic substance include quinolinol, benzoquinolinol, pyridylphenol, flavonol, hydroxyimidazopyridine, hydroxybenzazole, and hydroxytriazole. Particularly, a complex of an alkali metal and an organic substance is preferred, a complex of lithium and an organic substance is more preferred, and lithium quinolinol is especially preferred.

Examples of a method of forming each of the aforementioned layers constituting the light-emitting device include, but are not particularly limited to, resistance heating deposition, electron beam deposition, sputtering, a molecular lamination method, and a coating method, but usually, resistance heating deposition or electron beam deposition is preferable from the viewpoint of device property.

The thickness of the organic layer depends on the resistance value of an emissive substance and, therefore, it cannot be limited, but it is preferably 1 to 1000 nm. The film thickness of each of the emissive layer, the electron transporting layer and the hole transporting layer is preferably 1 nm or more and 200 nm or less, more preferably 5 nm or more and 100 nm or less.

The light-emitting device of the present invention has a function of being able to convert electric energy into light. Herein, a direct current is mainly used as the electric energy, but a pulse current or an alternate current can also be used. A current value and a voltage value are not particularly limited, but when the power consumed and life of the device are considered, they should be selected so that the maximum luminance is obtained by energy as low as possible.

The light-emitting device of the present invention is used suitably as a display that displays in a matrix and/or segment system.

In the matrix system, pixels for display are arranged two-dimensionally such as lattice-like arrangement or mosaic-like arrangement, and the collection of pixels displays letters and images. The shape and size of the pixel are determined depending on utility. For example, for displaying images and letters on personal computers, monitors and televisions, a square pixel being 300 µm or less at each side is usually used and, in the case of a large display such as a display panel, a pixel being millimeter order at each side is used. In the case of a monochromatic display, pixels having the same color may be arranged, and in the case of a color display, pixels having red, green and blue colors are arranged to perform display. In this case, typically, there are a delta type and a stripe type. A method of driving this matrix may be either a line sequential driving method or an active matrix. The line sequential driving has a simple structure, but when operation property is considered, the active matrix is more excellent in some cases, and it is necessary to use them properly depending on utility.

The segment system in the present invention is a system by which a pattern is formed so as to display predetermined information, and a region determined by arrangement of this pattern is made to emit light. Examples thereof include time and temperature displays in digital watches and thermometers, operating-state displays in audio equipment, IH cookers and so on, and panel displays of automobiles. The above-mentioned matrix display and segment display may exist together in the same panel.

The light-emitting device of the present invention can also be preferably used as backlight of various instruments. Backlight is used mainly for the purpose of improving the visibility of display apparatuses which do not emit light by themselves, and is used in liquid crystal display equipment, clocks, audio equipment, automobile panels, display panels, signs, and the like. In particular, the light-emitting device of the present invention is preferably used in backlight for liquid crystal display apparatuses, inter alia, for personal computers which are studied to be thinned, and can provide backlight thinner and lighter than conventional products.

### EXAMPLES

The present invention will be described by way of Examples, but the present invention is not limited thereto.

### Synthesis Example 1

### Synthesis of Compound [1]

Mixed were 26.0 g of bromofluoranthene, 35.2 g of bis (pinacolato) diboron, 27.2 g of potassium acetate and 462 mL of dimethylformamide, and the mixture was purged with nitrogen. To this mixed solution were added 0.75 g of a [1,1'-bis(diphenylphosphino)ferrocene]palladium (II) dichloride-dichloromethane complex, and the mixture was heated to 100°C. After 1 hour, the mixture was cooled to room temperature, 250 mL of ethyl acetate, 250 mL of toluene and 250 mL of water were then added, and the liquid was separated. The aqueous layer was extracted with 200 mL of ethyl acetate and 200 mL of toluene, and then combined with the foregoing organic layer, and the mixture was washed with 500 mL of water three times. The organic layer was dried over magnesium sulfate, and the solvent was distilled off. The resultant was purified by silica gel column chromatography, the eluate was evaporated, and vacuum-drying was performed to obtain 16.4 g of an intermediate A.

Next, 16.4 g of the intermediate A, 11.9 g of chloroiodobenzene, 251 mL of dimethoxy ethane and 67 ml of a 1.5 M aqueous sodium carbonate solution were mixed, and the mixture was purged with nitrogen. To this mixed solution was added 352 mg of bis(triphenylphosphine)palladium dichloride, and the mixture was heated and refluxed. After 3 hours, the mixture was cooled to room temperature, 250 ml of water was then added, and the precipitate was filtered, and dried by a vacuum drier. The product filtered was dissolved in toluene, activated carbon and QuadraSil (registered trademark) were then added, and the mixture was filtered with a silica pad. The solvent of the filtrate was distilled off, methanol was then added, and the precipitated solid was filtered, and dried. The resultant solid was re-crystallized with 100 mL of butyl acetate, filtered, and then vacuum-dried to obtain 8.4 g of a yellowish green solid of an intermediate B.

Next, 10.4 g of 5-aminoquinoline, 21.6 g of 2-bromoiodobenzene, 9.3 g of sodium-t-butoxide and 174 mL of toluene were mixed, and the mixture was purged with nitrogen. To this mixed solution were added 0.80 g of bis(dibenzylideneacetone)palladium (0) and 0.77 g of bis(diphenylphosphine)ferrocene, and the mixture was heated and refluxed. After 3 hours, the mixture was cooled to room temperature, and then filtered with celite, and the solvent of the filtrate was distilled off. The resultant was purified by silica gel column chromatography, the eluate was evaporated, and vacuum-drying was performed to obtain 19.4 g of an intermediate C.

Next, 19.4 g of the intermediate C, 9.6 g of potassium acetate and 82 mL of dimethylformamide were mixed, and the mixture was purged with nitrogen. To this mixed solution were added 0.29 g of palladium acetate and 0.85 g of triphenylphosphine, and the mixture was heated and refluxed. After 3 hours, the mixture was cooled to room temperature, and 300 mL of water was then added. An aqueous potassium hydroxide solution was added until the mixture became basic, and the precipitate was filtered, and dried by a vacuum drier. The resultant solid was re-crystallized with butyl acetate, filtered, and then vacuum-dried to obtain 11.0 g of an intermediate D (yield: 77%).

Next, 3.4 g of the intermediate B, 2.5 g of the intermediate D, 1.4 g of sodium-t-butoxide and 55 mL of o-xylene were mixed, and the mixture was purged with nitrogen. To this mixed solution were added 63 mg of bis (dibenzylideneacetone) palladium (0) and 100 mg of XPhos, and the mixture was heated and refluxed. After 3 hours, the mixture was cooled to room temperature, and then filtered, and the product filtered was washed with water. The product filtered was purified by silica gel column chromatography, and the eluate was evaporated. Methanol was added to precipitate a solid. Thereafter, the solid was filtered, and vacuum-dried to obtain 3.8 g of a yellowish green solid of a compound [1].

¹H-NMR analytical results of the resulting yellow solid are as follows, and it was confirmed that the resulting yellowish green solid was the compound [1].
Compound [1]: ¹H-NMR (CDCl₃(d=ppm)) δ 7.21(t,1H), 7.36-7.52(m,5H), 7.68-7.84(m,4H), 7.92-8.10(m,9H), 8.27(d,1H), 8.50(d,1H), 8.90(d,1H).

The compound [1] was used as a light-emitting device material after sublimation purification was performed at about 300°C under a pressure of 1 × 10⁻³ Pa using an oil diffusion pump. The HPLC purity (area % at a measurement wavelength of 254 nm) was 99.9% before sublimation purification, and 99.9% after sublimation purification.

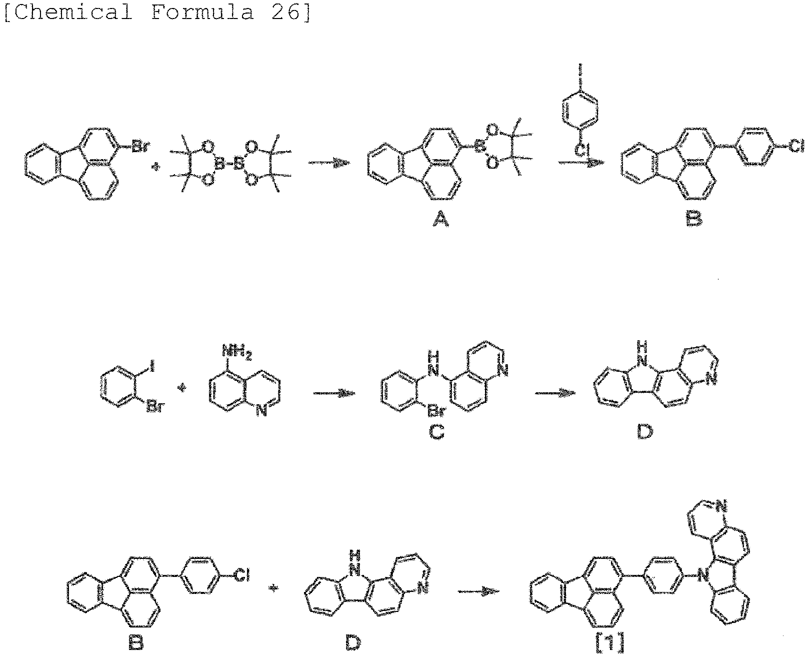

### Example 1

A glass substrate with an ITO transparent electroconductive film deposited thereon in a thickness of 165 nm (manufactured by GEOMATEC Co., Ltd., 11 Ω/□, sputtered product) was cut into 38 × 46 mm, and etched. The resulting substrate was ultrasonically washed with "SEMICOCLEAN 56" (trade name, manufactured by Furuuchi Chemical Corporation) for 15 minutes, and then washed with ultrapure water. This substrate was treated with UV-ozone for 1 hour immediately before preparation of a device, and placed in a vacuum deposition apparatus, and the air was evacuated until the degree of vacuum in the apparatus was 5 × 10⁻⁴ Pa or lower. By a resistance heating method, first, HAT-CN6 was deposited as a hole injection layer in a thickness of 5 nm, and HT-1 was deposited as a hole transporting layer in a thickness of 60 nm. Next, as an emissive layer, a host material H-1 and a dopant material D-1 were deposited in a thickness of 30 nm so that the doping concentration was 5% by weight. Next, as an electron transporting layer, the compound [1] was deposited and laminated in a thickness of 20 nm. Next, lithium fluoride was deposited in a thickness of 0.5 nm, and aluminum was then deposited in a thickness of 1000 nm to form a cathode, so that a 5 × 5 mm square device was prepared. The film thickness referred to herein is an indicated value on a crystal oscillation film thickness monitor. The properties of the light-emitting device at 1000 cd/m² included a driving voltage of 4.5 V and an external quantum efficiency of 4.4%. When the light-emitting device was driven at a constant current with the initial luminance set to 1000 cd/m², the luminance half-time at which the luminance decreased by 50% was 1200 hours. Compounds HAT-CN6, HT-1, H-1 and D-1 are the compounds shown below.

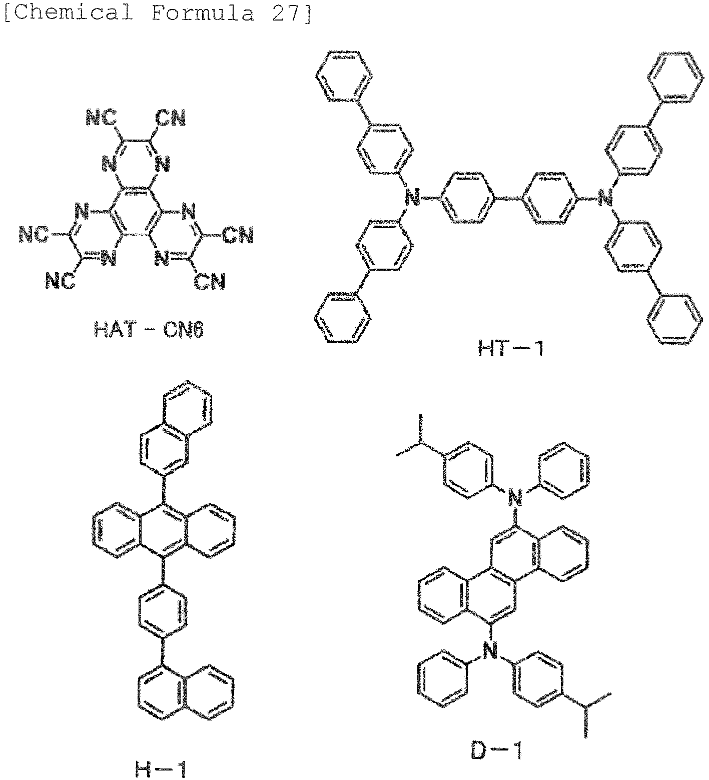

Examples 2 to 9. Example 4 is a reference example and does not form part of the present invention.

In the same manner as in Example 1 except that compounds described in Table 1 were used for the electron transporting layer, light-emitting devices were prepared and evaluated. The results are shown in Table 1. Compounds [2] to [9] are the compounds shown below. Compound [4] does not form part of the present invention.

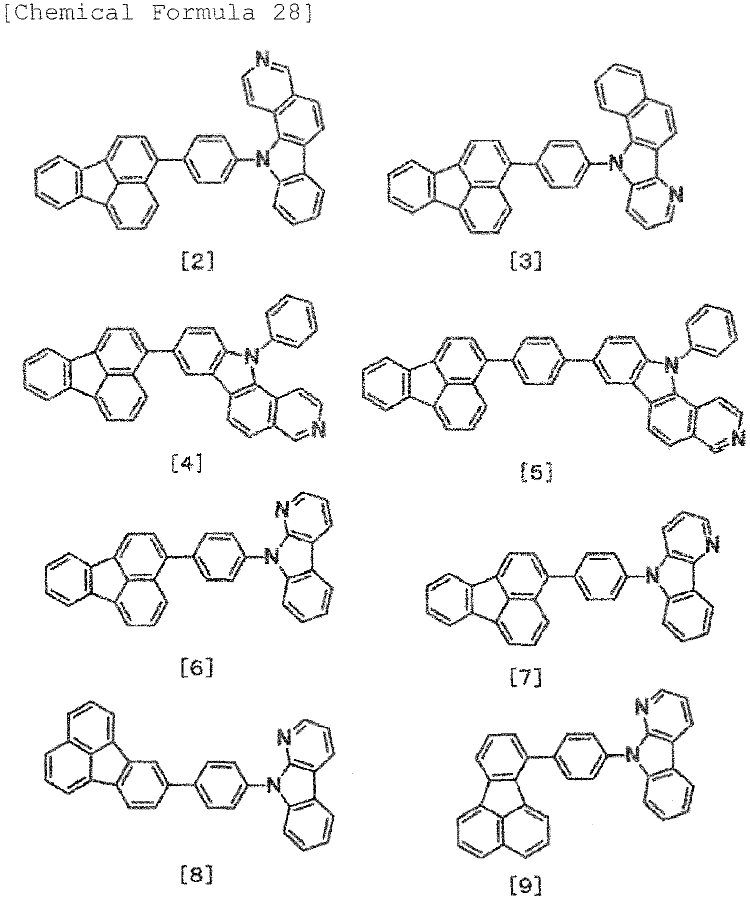

### Comparative Examples 1 to 3

In the same manner as in Example 1 except that compounds described in Table 1 were used for the electron transporting layer, light-emitting devices were prepared and evaluated. The results are shown in Table 1. E-1 to E-3 are the compounds shown below.

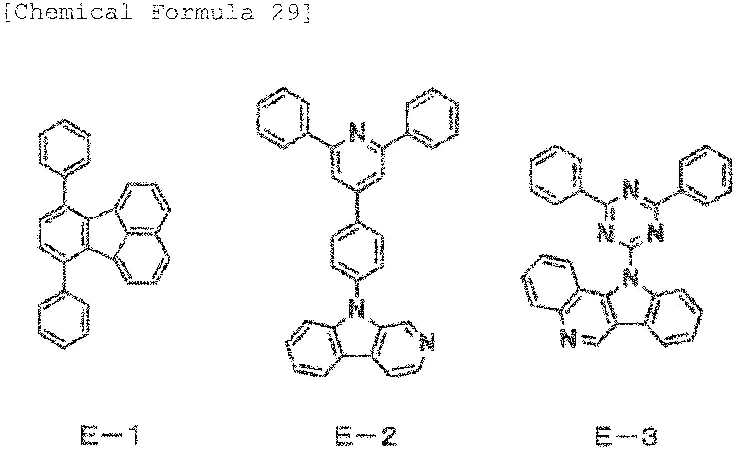

### Example 10

A glass substrate with an ITO transparent electroconductive film deposited thereon in a thickness of 165 nm (manufactured by GEOMATEC Co., Ltd., 11 Ω/□, sputtered product) was cut into 38 × 46 mm, and etched. The resulting substrate was ultrasonically washed with "SEMICOCLEAN 56" (trade name, manufactured by Furuuchi Chemical Corporation) for 15 minutes, and then washed with ultrapure water. This substrate was treated with UV-ozone for 1 hour immediately before preparation of a device, and placed in a vacuum deposition apparatus, and the air was evacuated until the degree of vacuum in the apparatus was 5 × 10⁻⁴ Pa or lower. By a resistance heating method, first, HAT-CN6 was deposited as a hole injection layer in a thickness of 5 nm, and HT-1 was deposited as a hole transporting layer in a thickness of 60 nm. Next, as an emissive layer, a host material H-1 and a dopant material D-1 were deposited in a thickness of 30 nm so that the doping concentration was 5% by weight. Next, as a first electron transporting layer, the compound [1] was deposited and laminated in a thickness of 10 nm. Further as a second electron transporting layer, the compound [1] used as an electron transporting material and cesium used as a donor material were laminated in a thickness of 10 nm so that the deposition rate ratio of the compound [1] and cesium was 20 : 1. Next, lithium fluoride was deposited in a thickness of 0.5 nm, and aluminum was then deposited in a thickness of 1000 nm to form a cathode, so that a 5 × 5 mm square device was prepared. The properties of the light-emitting device at 1000 cd/m² included a driving voltage of 4.3 V and an external quantum efficiency of 5.2%. When the light-emitting device was driven at a constant current with the initial luminance set to 1000 cd/m², the luminance half-time at which the luminance decreased by 50% was 1800 hours.

Examples 11 to 18. Example 13 is a reference example and does not form part of the present invention.

In the same manner as in Example 10 except that compounds described in Table 2 were used for the electron transporting layer, light-emitting devices were prepared and evaluated. The results are shown in Table 2.

### Comparative Examples 4 to 6

In the same manner as in Example 1 except that compounds described in Table 2 were used for the electron transporting layer, light-emitting devices were prepared and evaluated. The results are shown in Table 2.

### Example 19

A glass substrate with an ITO transparent electroconductive film deposited thereon in a thickness of 165 nm (manufactured by GEOMATEC Co., Ltd., 11 Ω/□, sputtered product) was cut into 38 × 46 mm, and etched. The resulting substrate was ultrasonically washed with "SEMICOCLEAN 56" (trade name, manufactured by Furuuchi Chemical Corporation) for 15 minutes, and then washed with ultrapure water. This substrate was treated with UV-ozone for 1 hour immediately before preparation of a device, and placed in a vacuum deposition apparatus, and the air was evacuated until the degree of vacuum in the apparatus was 5 × 10⁻⁴ Pa or lower. By a resistance heating method, first, HAT-CN6 was deposited as a hole injection layer in a thickness of 5 nm, and HT-1 was deposited as a hole transporting layer in a thickness of 60 nm. Next, as an emissive layer, a host material H-1 and a dopant material D-1 were deposited in a thickness of 30 nm so that the doping concentration was 5% by weight. Further as an electron transporting layer, the compound [1] used as an electron transporting material and 2E-1 used as a donor material were laminated in a thickness of 20 nm so that the deposition rate ratio of the compound [1] and 2E-1 was 1 : 1. This electron transporting layer is shown as a second electron transporting layer in Table 2. Next, lithium fluoride was deposited in a thickness of 0.5 nm, and aluminum was then deposited in a thickness of 1000 nm to form a cathode, so that a 5 × 5 mm square device was prepared. The properties of the light-emitting device at 1000 cd/m² included a driving voltage of 4.0 V and an external quantum efficiency of 5.7%. When the light-emitting device was driven at a constant current with the initial luminance set to 1000 cd/m², the luminance half-time at which the luminance decreased by 50% was 2100 hours.

Examples 20 to 27. Example 22 is a reference example and does not form part of the present invention.

In the same manner as in Example 19 except that compounds described in Table 2 were used for the electron transporting layer and the donor material, light-emitting devices were prepared and evaluated. The results are shown in Table 2. 2E-1 is the compound shown below.

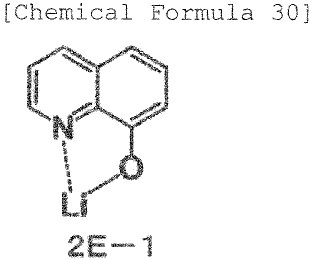

### Comparative Examples 7 to 9

In the same manner as in Example 11 except that compounds described in Table 2 were used for the electron transporting layer and the donor compound, light-emitting devices were prepared and evaluated. The results are shown in Table 2.

Example 28. Example 28 is a reference example and does not form part of the present invention.

A glass substrate with an ITO transparent electroconductive film deposited thereon in a thickness of 165 nm (manufactured by GEOMATEC Co., Ltd., 11 Ω/□, sputtered product) was cut into 38 × 46 mm, and etched. The resulting substrate was ultrasonically washed with "SEMICOCLEAN 56" (trade name, manufactured by Furuuchi Chemical Corporation) for 15 minutes, and then washed with ultrapure water. This substrate was treated with UV-ozone for 1 hour immediately before preparation of a device, and placed in a vacuum deposition apparatus, and the air was evacuated until the degree of vacuum in the apparatus was 5 × 10⁻⁴ Pa or lower. By a resistance heating method, first, HAT-CN6 was deposited as a hole injection layer in a thickness of 5 nm, and HT-1 was deposited as a hole transporting layer in a thickness of 60 nm. This hole transporting layer is shown as a first hole transporting layer in Table 3. Next, as an emissive layer, a host material H-2 and a dopant material D-2 were deposited in a thickness of 30 nm so that the doping concentration was 10% by weight. Next, as an electron transporting layer, the compound [4] was deposited and laminated in a thickness of 20 nm. Next, lithium fluoride was deposited in a thickness of 0.5 nm, and aluminum was then deposited in a thickness of 1000 nm to form a cathode, so that a 5 × 5 mm square device was prepared. The film thickness referred to herein was an indicated value on a crystal oscillation film thickness monitor. The properties of the light-emitting device at 4000 cd/m² included a driving voltage of 4.3 V and an external quantum efficiency of 11.9%. When the light-emitting device was driven at a constant current with the initial luminance set to 4000 cd/cm², the luminance half-time was 1100 hours. H-2 and D-2 are the compounds shown below.

[Chemical Formula 31] H-2 D-2

Example 29. Example 28 is a reference example and does not form part of the present invention.

A glass substrate with an ITO transparent electroconductive film deposited thereon in a thickness of 165 nm (manufactured by GEOMATEC Co., Ltd., 11 Ω/□, sputtered product) was cut into 38 × 46 mm, and etched. The resulting substrate was ultrasonically washed with "SEMICOCLEAN 56" (trade name, manufactured by Furuuchi Chemical Corporation) for 15 minutes, and then washed with ultrapure water. This substrate was treated with UV-ozone for 1 hour immediately before preparation of a device, and placed in a vacuum deposition apparatus, and the air was evacuated until the degree of vacuum in the apparatus was 5 × 10⁻⁴ Pa or lower. By a resistance heating method, first, HAT-CN6 was deposited as a hole injection layer in a thickness of 5 nm, and HT-1 was deposited as a hole transporting layer in a thickness of 50 nm. Further, HT-2 was deposited as a second hole transporting layer in a thickness of 10 nm. Next, as an emissive layer, a host material H-2 and a dopant material D-2 were deposited in a thickness of 30 nm so that the doping concentration was 10% by weight. Next, as an electron transporting layer, the compound [4] was deposited and laminated in a thickness of 20 nm. Next, lithium fluoride was deposited in a thickness of 0.5 nm, and aluminum was then deposited in a thickness of 1000 nm to form a cathode, so that a 5 × 5 mm square device was prepared. The film thickness referred to herein was an indicated value on a crystal oscillation film thickness monitor. The properties of the light-emitting device at 4000 cd/m² included a driving voltage of 4.3 V and an external quantum efficiency of 15.1%. When the light-emitting device was driven at a constant current with the initial luminance set to 4000 cd/cm², the luminance half-time was 1800 hours. HT-2 is the compound shown below.

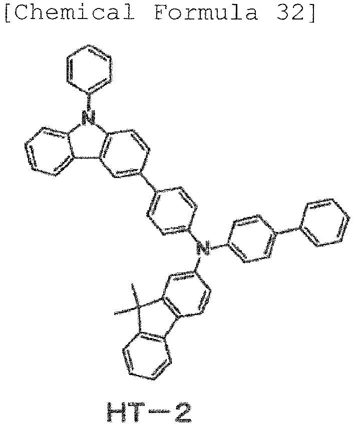

Examples 30 and 31. Examples 30 and 31 are reference examples and do not form part of the present invention.

In the same manner as in Example 29 except that compounds described in Table 3 were used for the second hole transporting layer, light-emitting devices were prepared and evaluated. The results are shown in Table 3. HT-3 and HT-4 are the compounds shown below.

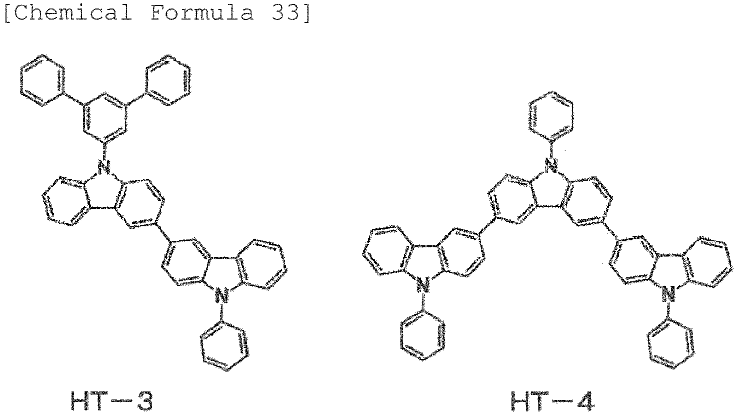

### Example 32

In the same manner as in Example 28 except that a compound [5] was used for the electron transporting layer, a light-emitting device was prepared and evaluated. The results are shown in Table 3.

### Examples 33 and 34

In the same manner as in Example 29 except that compounds described in Table 3 were used for the second hole transporting layer, and the compound [5] was used for the electron transporting layer, light-emitting devices were prepared and evaluated. The results are shown in Table 3.

### Comparative Examples 10 and 14

In the same manner as in Example 28 except that compounds described in Table 3 were used for the electron transporting layer, light-emitting devices were prepared and evaluated. The results are shown in Table 3.

### Comparative Examples 11 to 13 and 15 to 17

In the same manner as in Example 29 except that compounds described in Table 3 were used for the second hole transporting layer and the electron transporting layer, light-emitting devices were prepared and evaluated. The results are shown in Table 3.

**[Table 1]**

| | Emissive material | | | Electron transporting layer | Cathode | External quantum efficiency (%) | Driving voltage (V) | Luminance half-time (h) |
|---|---|---|---|---|---|---|---|---|
| | Host material | Dopant material | Emitted color | Compound | Metal | | | |
| Example 1 | H-1 | D-1 | Blue | Compound [1] | Al | 4.4 | 4.5 | 1200 |
| Example 2 | | | Blue | Compound [2] | Al | 4.4 | 4.4 | 1200 |
| Example 3 | | | Blue | Compound [3] | Al | 4.3 | 4.4 | 1300 |
| Example 4 | | | Blue | Compound [4] | Al | 4.9 | 4.1 | 2100 |
| Example 5 | | | Blue | Compound [5] | Al | 4.8 | 4.2 | 2000 |
| Example 6 | | | Blue | Compound [6] | Al | 4.0 | 4.7 | 1000 |
| Example 7 | | | Blue | Compound [7] | Al | 4.1 | 4.7 | 1100 |
| Example 8 | | | Blue | Compound [8] | Al | 3.8 | 4.9 | 800 |
| Example 9 | | | Blue | Compound [9] | Al | 3.7 | 4.9 | 800 |
| Comparative Example 1 | H-1 | D-1 | Blue | E-1 | Al | 2.8 | 8.0 | 300 |
| Comparative Example 2 | | | Blue | E-2 | Al | 2.9 | 6.7 | 300 |
| Comparative Example 3 | | | Blue | E-3 | Al | 2.7 | 6.8 | 300 |

**[Table 2]**

| | Emissive material | | | First electron transporting layer | Second electron transporting layer | | Cathode | External quantum efficiency (%) | Driving voltage (V) | Luminance half-time (h) |
|---|---|---|---|---|---|---|---|---|---|---|
| | Host material | Dopant material | Emitted color | Compound | Compound | Donor compound | Metal | | | |
| Example 10 | | | Blue | Compound [1] | Compound [1] | Cesium | Al | 5.2 | 4.3 | 1800 |
| Example 11 | | | Blue | Compound [2] | Compound [2] | Cesium | Al | 5.2 | 4.3 | 1800 |
| Example 12 | | | Blue | Compound [3] | Compound [3] | Cesium | Al | 5.1 | 4.2 | 1700 |
| Example 13 | | | Blue | Compound [4] | Compound [4] | Cesium | Al | 5.5 | 3.8 | 2600 |
| Example 14 | | | Blue | Compound [5] | Compound [5] | Cesium | Al | 5.4 | 3.9 | 2500 |
| Example 15 | | | Blue | Compound [6] | Compound [6] | Cesium | Al | 4.9 | 4.5 | 1500 |
| Example 16 | | | Blue | Compound [7] | Compound [7] | Cesium | Al | 4.8 | 4.5 | 1500 |
| Example 17 | | | Blue | Compound [8] | Compound [8] | Cesium | Al | 4.5 | 4.7 | 1100 |
| Example 18 | H-1 | D-1 | Blue | Compound [9] | Compound [9] | Cesium | Al | 4.4 | 4.7 | 1000 |
| Example 19 | | | Blue | None | Compound [1] | 2E-1 | Al | 5.7 | 4.0 | 2100 |
| Example 20 | | | Blue | None | Compound [2] | 2E-1 | Al | 5.6 | 4.1 | 2200 |
| Example 21 | | | Blue | None | Compound [3] | 2E-1 | Al | 5.7 | 4.0 | 2100 |
| Example 22 | | | Blue | None | Compound [4] | 2E-1 | Al | 6.3 | 3.7 | 3000 |
| Example 23 | | | Blue | None | Compound [5] | 2E-1 | Al | 6.2 | 3.7 | 3100 |
| Example 24 | | | Blue | None | Compound [6] | 2E-1 | Al | 5.2 | 4.2 | 1800 |
| Example 25 | | | Blue | None | Compound [7] | 2E-1 | Al | 5.3 | 4.1 | 1800 |
| Example 26 | | | Blue | None | Compound [8] | 2E-1 | Al | 4.9 | 4.5 | 1500 |
| Example 27 | | | Blue | None | Compound [9] | 2E-1 | Al | 4.9 | 4.5 | 1500 |
| Comparative Example 4 | H-1 | D-1 | Blue | E-1 | E-1 | Cesium | Al | 3.1 | 7.6 | 600 |
| Comparative Example 5 | | | Blue | E-2 | E-2 | Cesium | Al | 3.0 | 6.5 | 700 |
| Comparative Example 6 | | | Blue | E-3 | E-3 | Cesium | Al | 3.1 | 6.4 | 600 |
| Comparative Example 7 | | | Blue | None | E-1 | 2E-1 | Al | 4.1 | 7.2 | 800 |
| Comparative Example 8 | | | Blue | None | E-2 | 2E-1 | Al | 3.9 | 6.3 | 800 |
| Comparative Example 9 | | | Blue | None | E-3 | 2E-1 | Al | 4.1 | 6.2 | 900 |

**[Table 3]**

| | Hole injection layer | First hole transporting layer | Second hole transporting layer | Emissive layer | | Electron transporting layer | External quantum efficiency (%) | Driving voltage (V) | Luminance half-time (h) |
|---|---|---|---|---|---|---|---|---|---|
| | | | | Host material | Dopant material | | | | |
| Example 28 | HAT-CN6 | HT-1 | None | H-2 | D-2 | Compound [4] | 11.9 | 4.3 | 1100 |
| Example 29 | | | HT-2 | | | | 15.1 | 4.3 | 1800 |
| Example 30 | | | HT-3 | | | | 17.2 | 4.4 | 2500 |
| Example 31 | | | HT-4 | | | | 17.9 | 4.3 | 2400 |
| Example 32 | | | None | | | Compound [5] | 12.1 | 4.4 | 1200 |
| Example 33 | | | HT-2 | | | | 15.2 | 4.4 | 1700 |
| Example 34 | | | HT-3 | | | | 18.1 | 4.3 | 2600 |
| Example 35 | | | HT-4 | | | | 18.0 | 4.4 | 2500 |
| Comparative Example 10 | HAT-CN6 | HT-1 | None | H-2 | D-2 | E-1 | 6.0 | 8.1 | 200 |
| Comparative Example 11 | | | HT-2 | | | | 6.2 | 8.0 | 400 |
| Comparative Example 12 | | | HT-3 | | | | 6.5 | 8.0 | 700 |
| Comparative Example 13 | | | HT-4 | | | | 6.5 | 8.1 | 700 |
| Comparative Example 14 | | | None | | | E-2 | 7.1 | 6.3 | 300 |
| Comparative Example 15 | | | HT-2 | | | | 7.5 | 6.2 | 400 |
| Comparative Example 16 | | | HT-3 | | | | 8.1 | 6.3 | 800 |
| Comparative Example 17 | | | HT-4 | | | | 8.1 | 6.2 | 700 |

## Claims

1. A light-emitting device material comprising a compound represented by the following general formula (1):
[Chemical Formula 1]
Ar-L(̵Z)ₙ (1)
wherein Z is represented by the following general formula (2), and Ar is represented by the following general formula (3); L is a substituted or unsubstituted arylene group; n is 1 or 2; and when n is 2, two Zs may be the same or different; 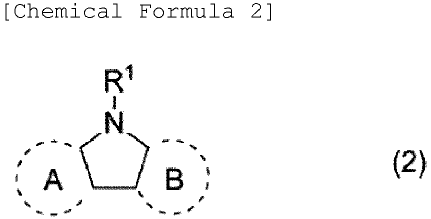 wherein ring A and ring B each represent a substituted or unsubstituted benzene ring, a substituted or unsubstituted fused aromatic hydrocarbon ring, a substituted or unsubstituted monocyclic aromatic heterocyclic ring, or a substituted or unsubstituted fused aromatic heterocyclic ring, with the proviso that at least one of atoms that form ring A and ring B is electron-accepting nitrogen; a substituent that ring A and ring B optionally have, and R¹ are each selected from the group consisting of an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, halogen, a carbonyl group, a carboxyl group, an oxycarbonyl group, a carbamoyl group and -P(=O)R²R³; R¹ may be hydrogen; R² and R³ are each an aryl group or a heteroaryl group; or R² and R³ may be fused to form a ring, with the proviso that the group is coupled to L at the position of any of R¹, ring A and ring B; and when n is 2, positions at which two Zs are coupled to L may be the same or different; 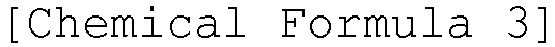 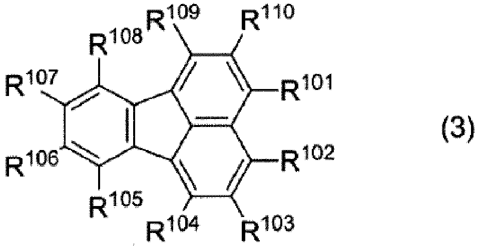 wherein R¹⁰¹ to R¹¹⁰ may be the same or different, and are each selected from the group consisting of hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, halogen, a carbonyl group, a carboxyl group, an oxycarbonyl group and a carbamoyl group; R¹⁰¹ to R¹¹⁰ may form a ring by adjacent substituents, with the proviso that R¹⁰¹ to R¹¹⁰ do not form a benzene ring; and the group is coupled to L at the position of any one of R¹⁰¹ to R¹¹⁰.

2. The light-emitting device material according to claim 1, wherein the compound represented by the general formula (1) is a compound represented by the following general formula (4): 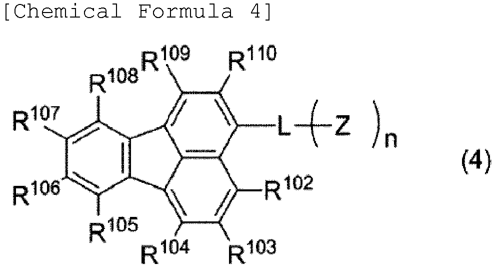 wherein R¹⁰² to R¹¹⁰ may be the same or different, and are each selected from the group consisting of hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, halogen, a carbonyl group, a carboxyl group, an oxycarbonyl group and a carbamoyl group; R¹⁰² to R¹¹⁰ may form a ring by adjacent substituents, with the proviso that R¹⁰² to R¹¹⁰ do not form a benzene ring; and L, Z and n are the same as in the general formula (1).

3. The light-emitting device material according to claim 1 or 2, wherein n is 1.

4. The light-emitting device material according to any one of claims 1 to 3, wherein Z is a group represented by any of the following general formulae (5) to (9): 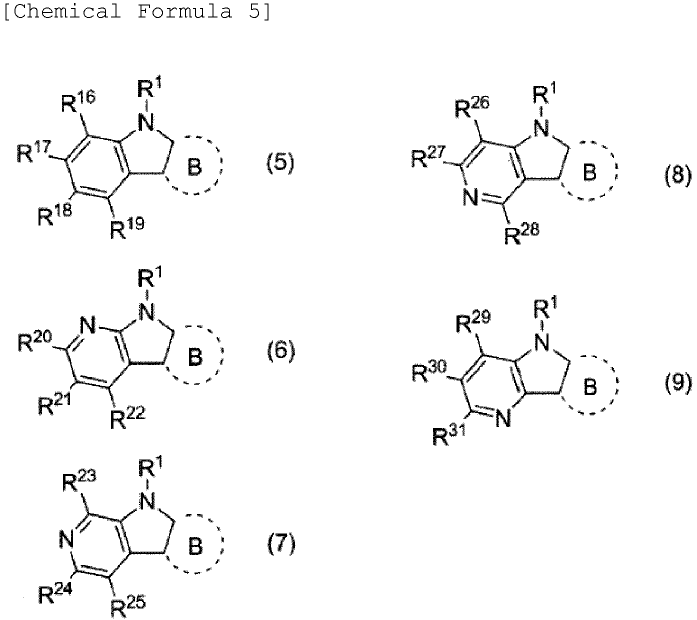 wherein ring B represents a substituted or unsubstituted benzene ring, a substituted or unsubstituted fused aromatic hydrocarbon ring, a substituted or unsubstituted monocyclic aromatic heterocyclic ring, or a substituted or unsubstituted fused aromatic heterocyclic ring, with the proviso that in the case of the general formula (5), ring B is a substituted or unsubstituted monocyclic aromatic heterocyclic ring, or a substituted or unsubstituted fused aromatic heterocyclic ring, and at least one of atoms that form ring B is electron-accepting nitrogen; a substituent that ring B optionally has, and R¹ are the same as in the general formula (2); and R¹⁶ to R³¹ are each selected from the group consisting of hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, halogen, a carbonyl group, a carboxyl group, an oxycarbonyl group, a carbamoyl group and - P(=O)R²R³, with the proviso that the group is coupled to L at the position of any of R¹, R¹⁶ to R¹⁹ and ring B in the case of the general formula (5); at the position of any of R¹, R²¹ to R²² and ring B in the case of the general formula (6); at the position of any of R¹, R²³ to R²⁵ and ring B in the case of the general formula (7); at the position of any of R¹, R²⁶ to R²⁸ and ring B in the case of the general formula (8); and at the position of any of R¹, R²⁹ to R³¹ and ring B in the case of the general formula (9); and when n is 2, two Zs may be the same or different.

5. The light-emitting device material according to claim 4, wherein ring B is a structure represented by any of the following general formulae (10) to (13): 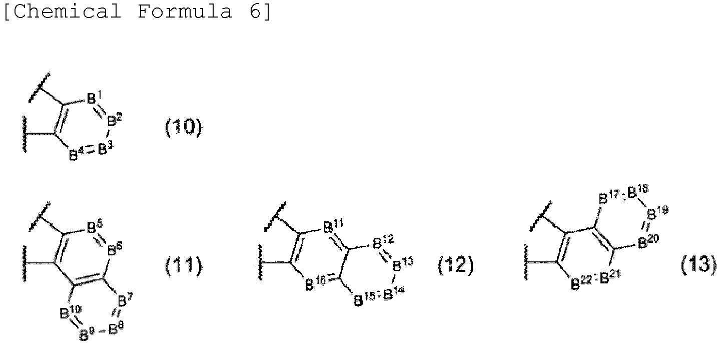 wherein B¹ to B²² represent C-R³² or N, with the proviso that when Z is a group represented by the general formula (5), at least one of B^{k}s (K = 1 to 22) contained in ring B is electron-accepting nitrogen; a substituent that B¹ to B²² optionally have is the same as in the general formula (2); and R³² is selected from the group consisting of hydrogen, an alkyl group, a cycloalkyl group, a heterocyclic group, an alkenyl group, a cycloalkenyl group, an alkynyl group, an alkoxy group, an alkylthio group, an aryl ether group, an aryl thioether group, an aryl group, a heteroaryl group, halogen, a carbonyl group, a carboxyl group, an oxycarbonyl group, a carbamoyl group and - P(=O)R²R³.

6. The light-emitting device material according to any one of claims 1 to 4, wherein Z is coupled to L at the position of any of ring A and ring B when Z is represented by the general formula (2); at the position of any of R¹⁶ to R¹⁹ and ring B when Z is represented by the general formula (5); at the position of any of R²⁰ to R²² and ring B when Z is represented by the general formula (6); at the position of any of R²³ to R²⁵ and ring B when Z is represented by the general formula (7); at the position of any of R²⁶ to R²⁸ and ring B when Z is represented by the general formula (8); and at the position of any of R²⁹ to R³¹ and ring B when Z is represented by the general formula (9).

7. A light-emitting device which has an organic layer between an anode and a cathode and emits light by means of electric energy, wherein the light-emitting device contains the light-emitting device material according to any one of claims 1 to 6 in the organic layer.

8. The light-emitting device according to claim 7, wherein the organic layer comprises an electron transporting layer, and the electron transporting layer comprises the light-emitting device material according to any of claims 1 to 6.

9. The light-emitting device according to claim 8, wherein the electron transporting layer further comprises a donor compound.

10. The light-emitting device according to claim 9, wherein the donor compound is an alkali metal, an inorganic salt containing an alkali metal, a complex of an alkali metal and an organic substance, an alkaline earth metal, an inorganic salt containing an alkaline earth metal, or a complex of an alkaline earth metal and an organic substance.

11. The light-emitting device according to claim 10, wherein the donor compound is a complex of an alkali metal and an organic substance or a complex of an alkaline earth metal and an organic substance.

12. The light-emitting device according to any one of claims 7 to 11, further comprising a hole transporting layer between the anode and the cathode, wherein the hole transporting layer contains a material having a carbazole skeleton.

13. The light-emitting device according to claim 12, wherein the material having a carbazole skeleton is a carbazole polymer.

## Patentansprüche

1. Material für eine lichtemittierende Vorrichtung, umfassend eine Verbindung, die durch die folgende allgemeine Formel dargestellt ist:
[Chemische Formel 1]
Ar-L(̵Z)ₙ (1)
wobei Z durch die folgende allgemeine Formel (2) dargestellt ist, und Ar durch die folgende allgemeine Formel (3) dargestellt ist; L eine substituierte oder unsubstituierte Arylengruppe ist; n gleich 1 oder 2 ist; und wenn n gleich 2 ist, zwei Z gleich oder verschieden sein können; 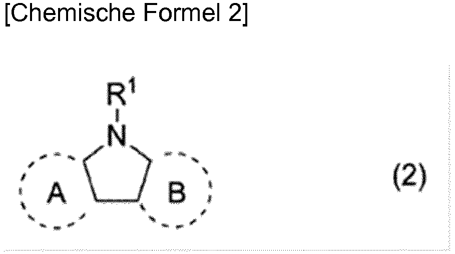 wobei ein Ring A und ein Ring B jeweils einen substituierten oder unsubstituierten Benzolring, einen substituierten oder unsubstituierten kondensierten aromatischen Kohlenwasserstoffring, einen substituierten oder unsubstituierten monocyclischen aromatischen heterocyclischen Ring, oder einen substituierten oder unsubstituierten kondensierten aromatischen heterocyclischen Ring mit der Maßgabe darstellen, dass zumindest eines der Atome, die den Ring A und den Ring B bilden, elektronenaufnehmender Stickstoff ist; ein Substituent, den Ring A und Ring B gegebenenfalls aufweisen, und R¹ jeweils ausgewählt sind aus der Gruppe, bestehend aus einer Alkylgruppe, einer Cycloalkylgruppe, einer heterocyclischen Gruppe, einer Alkenylgruppe, einer Cycloalkenylgruppe, einer Alkinylgruppe, einer Alkoxygruppe, einer Alkylthiogruppe, einer Arylethergruppe, einer Arylthioethergruppe, einer Arylgruppe, einer Heteroarylgruppe, einem Halogen, einer Carbonylgruppe, einer Carboxylgruppe, einer Oxycarbonylgruppe, einer Carbamoylgruppe und -P(=O)R²R³; R¹ Wasserstoff sein kann; R² und R³ jeweils eine Arylgruppe oder eine Heteroarylgruppe sind; oder R² und R³ kondensiert sein können, um einen Ring mit der Maßgabe zu bilden, dass die Gruppe an der Position von R¹, Ring A oder Ring B an L gekoppelt ist; und wenn n gleich 2 ist, Positionen, an denen zwei Z an L gekoppelt sind, gleich oder verschieden sein können; 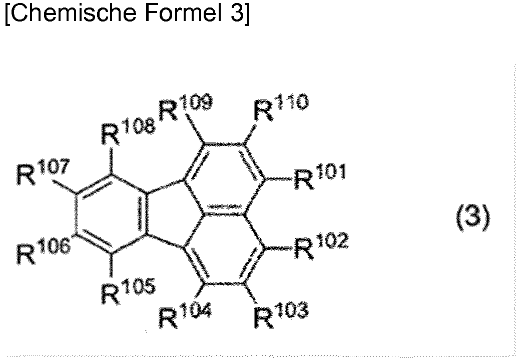 wobei R¹⁰¹ bis R¹¹⁰ gleich oder verschieden sein können und jeweils ausgewählt sind aus der Gruppe, bestehend aus Wasserstoff, einer Alkylgruppe, einer Cycloalkylgruppe, einer heterocyclischen Gruppe, einer Alkenylgruppe, einer Cycloalkenylgruppe, einer Alkinylgruppe, einer Alkoxygruppe, einer Alkylthiogruppe, einer Arylethergruppe, einer Arylthioethergruppe, einer Arylgruppe, einer Heteroarylgruppe, einem Halogen, einer Carbonylgruppe, einer Carboxylgruppe, einer Oxycarbonylgruppe und einer Carbamoylgruppe; R¹⁰¹ bis R¹¹⁰ durch benachbarte Substituenten einen Ring mit der Maßgabe bilden können, dass R¹⁰¹ bis R¹¹⁰ keinen Benzolring bilden; und die Gruppe an der Position eines beliebigen von R¹⁰¹ bis R¹¹⁰ an L gekoppelt ist.

2. Material für eine lichtemittierende Vorrichtung nach Anspruch 1, wobei die durch die allgemeine Formel (1) dargestellte Verbindung eine durch die folgende allgemeine Formel (4) dargestellte Verbindung ist: 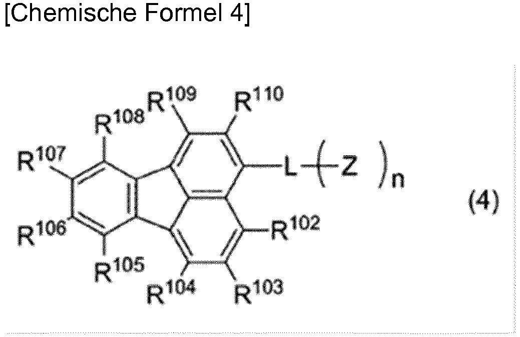 wobei R¹⁰² bis R¹¹⁰ gleich oder verschieden sein können und jeweils ausgewählt sind aus der Gruppe, bestehend aus Wasserstoff, einer Alkylgruppe, einer Cycloalkylgruppe, einer heterocyclischen Gruppe, einer Alkenylgruppe, einer Cycloalkenylgruppe, einer Alkinylgruppe, einer Alkoxygruppe, einer Alkylthiogruppe, einer Arylethergruppe, einer Arylthioethergruppe, einer Arylgruppe, einer Heteroarylgruppe, einem Halogen, einer Carbonylgruppe, einer Carboxylgruppe, einer Oxycarbonylgruppe und einer Carbamoylgruppe; R¹⁰² bis R¹¹⁰ durch benachbarte Substituenten einen Ring mit der Maßgabe bilden können, dass R¹⁰² bis R¹¹⁰ keinen Benzolring bilden; und L, Z und n die gleichen wie in der allgemeinen Formel (1) sind.

3. Material für eine lichtemittierende Vorrichtung nach Anspruch 1 oder 2, wobei n gleich 1 ist.

4. Material für eine lichtemittierende Vorrichtung nach einem der Ansprüche 1 bis 3, wobei Z eine Gruppe ist, die durch eine der folgenden allgemeinen Formeln (5) bis (9) dargestellt ist: 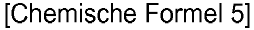 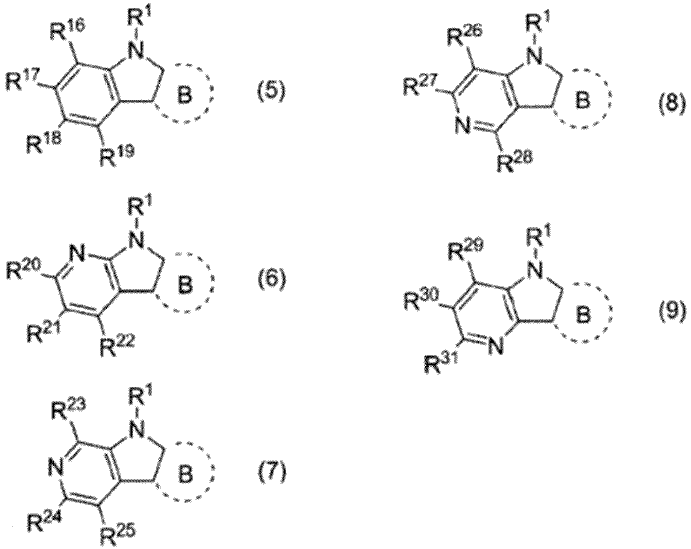 wobei der Ring B einen substituierten oder unsubstituierten Benzolring, einen substituierten oder unsubstituierten kondensierten aromatischen Kohlenwasserstoffring, einen substituierten oder unsubstituierten monocyclischen aromatischen heterocyclischen Ring oder einen substituierten oder unsubstituierten kondensierten aromatischen heterocyclischen Ring mit der Maßgabe darstellt, dass im Fall der allgemeinen Formel (5) der Ring B ein substituierter oder unsubstituierter monocyclischer aromatischer heterocyclischer Ring oder ein substituierter oder unsubstituierter kondensierter aromatischer heterocyclischer Ring ist und zumindest eines der Atome, die den Ring B bilden, elektronenaufnehmender Stickstoff ist; ein Substituent, den Ring B und R¹ gegebenenfalls aufweisen, die gleichen wie in der allgemeinen Formel (2) sind; und R¹⁶ bis R³¹ jeweils ausgewählt sind aus der Gruppe, bestehend aus Wasserstoff, einer Alkylgruppe, einer Cycloalkylgruppe, einer heterocyclischen Gruppe, einer Alkenylgruppe, einer Cycloalkenylgruppe, einer Alkinylgruppe, einer Alkoxygruppe, einer Alkylthiogruppe, einer Arylethergruppe einer Arylthioethergruppe, einer Arylgruppe, einer Heteroarylgruppe, einem Halogen, einer Carbonylgruppe, einer Carboxylgruppe, einer Oxycarbonylgruppe, einer Carbamoylgruppe und - P(=O)R²R³, mit der Maßgabe, dass die Gruppe an der Position eines beliebigen von R¹, R¹⁶ bis R¹⁹ und des Rings B im Fall der allgemeinen Formel (5); an der Position eines beliebigen von R¹, R²⁰ bis R²² und des Rings B im Fall der allgemeinen Formel (6); an der Position eines beliebigen von R¹, R²³ bis R²⁵ und des Rings B im Fall der allgemeinen Formel (7); an der Position eines beliebigen von R¹, R²⁶ bis R²⁸ und des Rings B im Fall der allgemeinen Formel (8); und an der Position eines beliebigen von R¹, R²⁹ bis R³¹ und des Rings B im Fall der allgemeinen Formel (9) an L gekoppelt ist; und wenn n gleich 2 ist, zwei Z gleich oder verschieden sein können.

5. Material für eine lichtemittierende Vorrichtung nach Anspruch 4, wobei der Ring B eine Struktur ist, die durch eine der folgenden allgemeinen Formeln (10) bis (13) dargestellt ist: 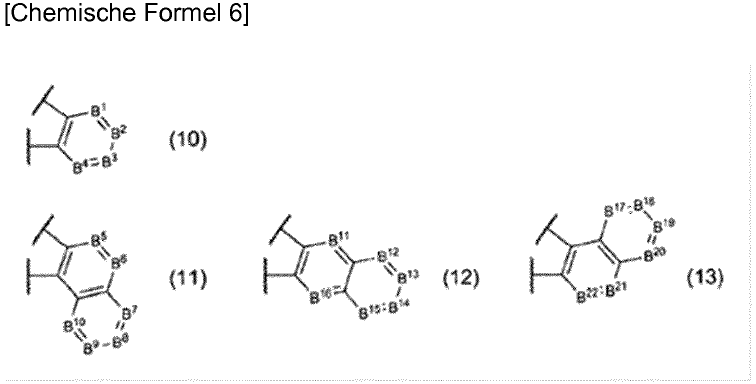 wobei B¹ bis B²² C-R³² oder N mit der Maßgabe darstellen, dass, wenn Z eine durch die allgemeine Formel (5) dargestellte Gruppe ist, zumindest eines der im Ring B enthaltenen B^{k}s (K = 1 bis 22) elektronenaufnehmender Stickstoff ist; ein Substituent, den B¹ bis B²² gegebenenfalls aufweisen, der gleiche wie in der allgemeinen Formel (2) ist; und R³² ausgewählt ist aus der Gruppe, bestehend aus Wasserstoff, einer Alkylgruppe, einer Cycloalkylgruppe, einer heterocyclischen Gruppe, einer Alkenylgruppe, einer Cycloalkenylgruppe, einer Alkinylgruppe, einer Alkoxygruppe, einer Alkylthiogruppe, einer Arylethergruppe, einer Arylthioethergruppe, einer Arylgruppe, einer Heteroarylgruppe, einem Halogen, einer Carbonylgruppe, einer Carboxylgruppe, einer Oxycarbonylgruppe, einer Carbamoylgruppe und-P(=O)R²R³.

6. Material für eine lichtemittierende Vorrichtung nach einem der Ansprüche 1 bis 4, wobei Z an der Position von Ring A oder Ring B, wenn Z durch die allgemeine Formel (2) dargestellt ist; an der Position eines beliebigen von R¹⁶ bis R¹⁹ und des Rings B, wenn Z durch die allgemeine Formel (5) dargestellt ist; an der Position eines beliebigen von R²⁰ bis R²² und des Rings B, wenn Z durch die allgemeine Formel (6) dargestellt ist; an der Position eines beliebigen von R²³ bis R²⁵ und des Rings B, wenn Z durch die allgemeine Formel (7) dargestellt ist; an der Position eines beliebigen von R²⁶ bis R²⁸ und des Rings B, wenn Z durch die allgemeine Formel (8) dargestellt ist; und an der Position eines beliebigen von R²⁹ bis R³¹ und des Rings B an L gekoppelt ist, wenn Z durch die allgemeine Formel (9) dargestellt ist.

7. Lichtemittierende Vorrichtung, die eine organische Schicht zwischen einer Anode und einer Kathode aufweist und Licht mittels elektrischer Energie emittiert, wobei die lichtemittierende Vorrichtung das Material für eine lichtemittierende Vorrichtung nach einem der Ansprüche 1 bis 6 in der organischen Schicht enthält.

8. Lichtemittierende Vorrichtung nach Anspruch 7, wobei die organische Schicht eine Elektronentransportschicht umfasst, und die Elektronentransportschicht das Material für eine lichtemittierende Vorrichtung nach einem der Ansprüche 1 bis 6 umfasst.

9. Lichtemittierende Vorrichtung nach Anspruch 8, wobei die Elektronentransportschicht ferner eine Donorverbindung umfasst.

10. Lichtemittierende Vorrichtung nach Anspruch 9, wobei die Donorverbindung ein Alkalimetall, ein anorganisches Salz, das ein Alkalimetall enthält, ein Komplex aus einem Alkalimetall und einer organischen Substanz, ein Erdalkalimetall, ein anorganisches Salz, das ein Erdalkalimetall enthält, oder ein Komplex aus einem Erdalkalimetall und einer organischen Substanz ist.

11. Lichtemittierende Vorrichtung nach Anspruch 10, wobei die Donorverbindung ein Komplex aus einem Alkalimetall und einer organischen Substanz, oder ein Komplex aus einem Erdalkalimetall und einer organischen Substanz ist.

12. Lichtemittierende Vorrichtung nach einem der Ansprüche 7 bis 11, ferner umfassend eine Lochtransportschicht zwischen der Anode und der Kathode, wobei die Lochtransportschicht ein Material mit einem Carbazolgerüst enthält.

13. Lichtemittierende Vorrichtung nach Anspruch 12, wobei das Material mit einem Carbazolgerüst ein Carbazolpolymer ist.

## Revendications

1. Matériau pour dispositif d'émission de lumière comprenant un composé représenté par la formule générale (1) suivante :
[Formule Chimique 1]
Ar-L(̵Z)ₙ (1)
dans laquelle Z est représenté par la formule générale (2) suivante et Ar est représenté par la formule générale (3) suivante ; L représente un groupe arylène substitué ou non substitué ; n vaut 1 ou 2 ; et lorsque n vaut 2, deux Z peuvent être identiques ou différents ; 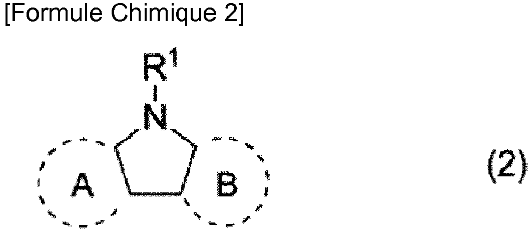 dans laquelle le noyau A et le noyau B représentent chacun un noyau benzène substitué ou non substitué, un noyau hydrocarboné aromatique condensé substitué ou non substitué, un noyau hétérocyclique aromatique monocyclique substitué ou non substitué, ou un noyau hétérocyclique aromatique condensé substitué ou non substitué, à condition qu'au moins l'un des atomes qui forment le noyau A et le noyau B soit un azote accepteur d'électrons ; un substituant que possèdent éventuellement le noyau A et le noyau B, et R¹ sont chacun choisis dans le groupe constitué par un groupe alkyle, un groupe cycloalkyle, un groupe hétérocyclique, un groupe alcényle, un groupe cycloalcényle, un groupe alcynyle, un groupe alcoxy, un groupe alkylthio, un groupe aryléther, un groupe arylthioéther, un groupe aryle, un groupe hétéroaryle, un halogène, un groupe carbonyle, un groupe carboxyle, un groupe oxycarbonyle, un groupe carbamoyle et -P(=0)R²R³; R¹ peut être un hydrogène ; R² et R³ représentent chacun un groupe aryle ou un groupe hétéroaryle ; ou R² et R³ peuvent être condensés pour former un noyau, à condition que le groupe soit couplé à L au niveau de la position de l'un de R¹, du noyau A et du noyau B ; et lorsque n vaut 2, les positions au niveau desquelles deux Z sont couplés à L peuvent être identiques ou différentes ; 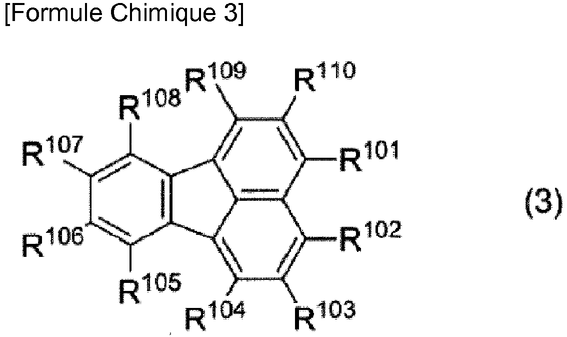 dans laquelle R¹⁰¹ à R¹¹⁰ peuvent être identiques ou différents, et sont chacun choisis dans le groupe constitué par un hydrogène, un groupe alkyle, un groupe cycloalkyle, un groupe hétérocyclique, un groupe alcényle, un groupe cycloalcényle, un groupe alcynyle, un groupe alcoxy, un groupe alkylthio, un groupe aryléther, un groupe arylthioéther, un groupe aryle, un groupe hétéroaryle, un halogène, un groupe carbonyle, un groupe carboxyle, un groupe oxycarbonyle et un groupe carbamoyle ; R¹⁰¹ à R¹¹⁰ peuvent former un noyau par des substituants adjacents, à condition que R¹⁰¹ à R¹¹⁰ ne forment pas de noyau benzène ; et le groupe est couplé à L au niveau de la position de l'un quelconque de R¹⁰¹ à R¹¹⁰.

2. Matériau pour dispositif d'émission de lumière selon la revendication 1, dans lequel le composé représenté par la formule générale (1) est un composé représenté par la formule générale (4) suivante : 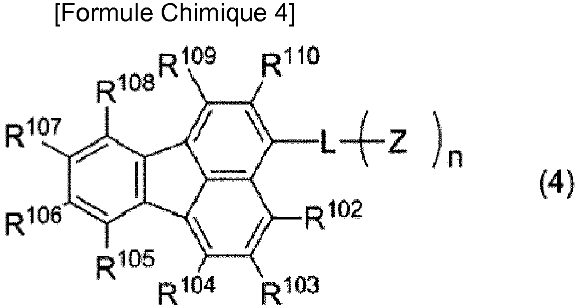 dans laquelle R¹⁰² à R¹¹⁰ peuvent être identiques ou différents et sont chacun choisis dans le groupe constitué par un hydrogène, un groupe alkyle, un groupe cycloalkyle, un groupe hétérocyclique, un groupe alcényle, un groupe cycloalcényle, un groupe alcynyle, un groupe alcoxy, un groupe alkylthio, un groupe aryléther, un groupe arylthioéther, un groupe aryle, un groupe hétéroaryle, un halogène, un groupe carbonyle, un groupe carboxyle, un groupe oxycarbonyle et un groupe carbamoyle ; R¹⁰² à R¹¹⁰ peuvent former un noyau par des substituants adjacents, à condition que R¹⁰² à R¹¹⁰ ne forment pas de noyau benzène ; et L, Z et n sont identiques à ceux de la formule générale (1).

3. Matériau pour dispositif d'émission de lumière selon la revendication 1 ou 2, dans lequel n vaut 1.

4. Matériau pour dispositif d'émission de lumière selon l'une quelconque des revendications 1 à 3, dans lequel Z est un groupe représenté par l'une des formules générales (5) à (9) suivantes : 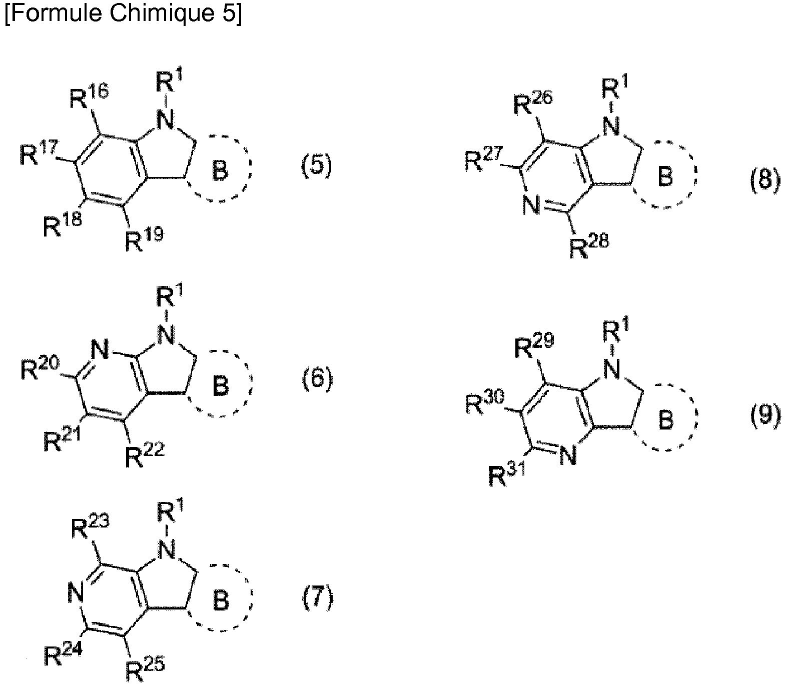 où le noyau B représente un noyau benzène substitué ou non substitué, un noyau hydrocarboné aromatique condensé substitué ou non substitué, un noyau hétérocyclique aromatique monocyclique substitué ou non substitué ou un noyau hétérocyclique aromatique condensé substitué ou non substitué, à condition que, dans le cas de la formule générale (5), le noyau B est un noyau hétérocyclique aromatique monocyclique substitué ou non substitué, ou un noyau hétérocyclique aromatique condensé substitué ou non substitué, et au moins l'un des atomes qui forment le noyau B est un azote accepteur d'électrons ; un substituant que possède éventuellement le noyau B, et R¹ sont identiques à ceux de la formule générale (2) ; et R¹⁶ à R³¹ sont chacun choisis dans le groupe constitué par un hydrogène, un groupe alkyle, un groupe cycloalkyle, un groupe hétérocyclique, un groupe alcényle, un groupe cycloalcényle, un groupe alcynyle, un groupe alcoxy, un groupe alkylthio, un groupe aryléther, un groupe arylthioéther, un groupe aryle, un groupe hétéroaryle, un halogène, un groupe carbonyle, un groupe carboxyle, un groupe oxycarbonyle, un groupe carbamoyle et -P(=O)R²R³, à condition que le groupe soit couplé à L au niveau de la position de l'un de R¹, R¹⁶ à R¹⁹ et du noyau B dans le cas de la formule générale (5) ; au niveau de la position de l'un de R¹, R²⁰ à R²² et du noyau B dans le cas de la formule générale (6) ; au niveau de la position de l'un de R¹, R²³ à R²⁵ et du noyau B dans le cas de la formule générale (7) ; au niveau de la position de l'un de R¹, R²⁶ à R²⁸ et du noyau B dans le cas de la formule générale (8) ; et au niveau de la position de l'un de R¹, R²⁹ à R³¹ et du noyau B dans le cas de la formule générale (9) ; et lorsque n vaut 2, deux Z peuvent être identiques ou différents.

5. Matériau pour dispositif d'émission de lumière selon la revendication 4, dans lequel le noyau B est une structure représentée par l'une des formules générales (10) à (13) suivantes : 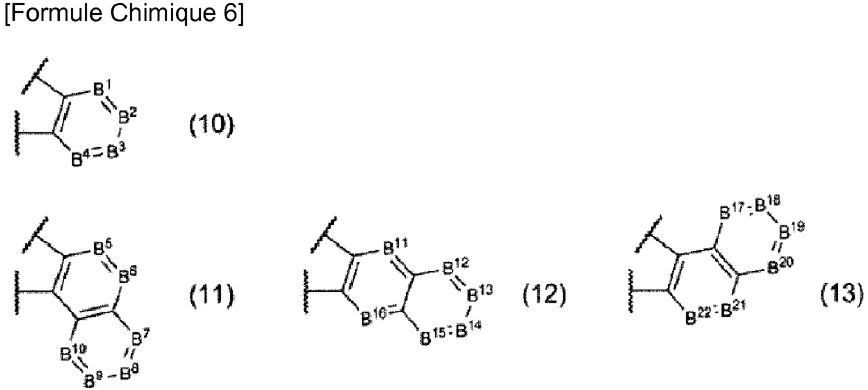 où B¹ à B²² représentent C-R³² ou N, à condition que lorsque Z est un groupe représenté par la formule générale (5), au moins l'un des B^{k} (K = 1 à 22) contenus dans le noyau B soit un azote accepteur d'électrons ; un substituant que possèdent éventuellement B¹ à B²² est identique à celui de la formule générale (2) ; et R³² est choisi dans le groupe constitué par un hydrogène, un groupe alkyle, un groupe cycloalkyle, un groupe hétérocyclique, un groupe alcényle, un groupe cycloalcényle, un groupe alcynyle, un groupe alcoxy, un groupe alkylthio, un groupe aryléther, un groupe arylthioéther, un groupe aryle, un groupe hétéroaryle, un halogène, un groupe carbonyle, un groupe carboxyle, un groupe oxycarbonyle, un groupe carbamoyle et -P(=O)R²R³.

6. Matériau pour dispositif d'émission de lumière selon l'une quelconque des revendications 1 à 4, dans lequel Z est couplé à L au niveau de la position de l'un des noyaux A et B lorsque Z est représenté par la formule générale (2) ; au niveau de la position de l'un de R¹⁶ à R¹⁹ et du noyau B lorsque Z est représenté par la formule générale (5) ; au niveau de la position de l'un de R²⁰ à R²² et du noyau B lorsque Z est représenté par la formule générale (6) ; au niveau de la position de l'un de R²³ à R²⁵ et du noyau B lorsque Z est représenté par la formule générale (7) ; au niveau de la position de l'un de R²⁶ à R²⁸ et du noyau B lorsque Z est représenté par la formule générale (8) ; et au niveau de la position de l'un de R²⁹ à R³¹ et du noyau B lorsque Z est représenté par la formule générale (9).

7. Dispositif d'émission de lumière qui a une couche organique entre une anode et une cathode et qui émet de la lumière à l'aide de l'énergie électrique, dans lequel le dispositif d'émission de lumière contient le matériau pour dispositif d'émission de lumière selon l'une quelconque des revendications 1 à 6 dans la couche organique.

8. Dispositif d'émission de lumière selon la revendication 7, dans lequel la couche organique comprend une couche de transport d'électrons et la couche de transport d'électrons comprend le matériau pour dispositif d'émission de lumière selon l'une des revendications 1 à 6.

9. Dispositif d'émission de lumière selon la revendication 8, dans lequel la couche de transport d'électrons comprend en outre un composé donneur.

10. Dispositif d'émission de lumière selon la revendication 9, dans lequel le composé donneur est un métal alcalin, un sel inorganique contenant un métal alcalin, un complexe d'un métal alcalin et d'une substance organique, un métal alcalino-terreux, un sel inorganique contenant un métal alcalino-terreux, ou un complexe d'un métal alcalino-terreux et d'une substance organique.

11. Dispositif d'émission de lumière selon la revendication 10, dans lequel le composé donneur est un complexe d'un métal alcalin et d'une substance organique ou un complexe d'un métal alcalino-terreux et d'une substance organique.

12. Dispositif d'émission de lumière selon l'une quelconque des revendications 7 à 11, comprenant en outre une couche de transport de trous entre l'anode et la cathode, dans lequel la couche de transport de trous contient un matériau ayant un squelette carbazole.

13. Dispositif d'émission de lumière selon la revendication 12, dans lequel le matériau ayant un squelette carbazole est un polymère de carbazole.
